# EUROPEAN PATENT SPECIFICATION

(11) **EP 2 027 840 B1**
(45) Date of publication and mention of the grant of the patent: **13.04.2016**
(21) Application number: 07743976.8
(22) Date of filing: 23.05.2007
(51) Int. Cl.: A61F 13/15, A61F 13/49, A61F 13/491

(54) **DISPOSABLE DIAPER**
EINWEGWINDEL
COUCHE JETABLE

(30) Priority: 15.06.2006 JP 2006166470
(43) Date of publication of application: 25.02.2009
(73) Proprietor: Uni-Charm Corporation, Ehime 799-0111 (JP)
(72) Inventor: SASAYAMA, Kenichi, Kanonji-shi, Kagawa 769-1602 (JP)
(74) Representative: Dolleymores
(86) International application number: PCT/JP2007/060542
(87) International publication number: WO 2007/145061

(56) References cited:
- WO-A1-98/43574
- JP-A- 11 043 802
- JP-A- 2002 282 292
- JP-A- 2005 080 996
- JP-A- 2005 080 996
- JP-A- 2005 198 900
- US-A1- 2008 140 042

## Description

### TECHNICAL FIELD

The present invention relates to disposable diapers, and more particularly, to disposable diapers having an opening through which the male sex organ can be drawn out.

### BACKGROUND ART

Conventionally, absorbent articles for use by elderly persons, physically disabled, etc., include, for example, disposable diapers, sweat pants, and incontinence pants has been proposed. The absorbent articles being properly used in accordance with a wearer's lifestyle and care level. Typically, due to their features of maintaining and absorbing excrement without leakage, disposable diapers are used as auxiliary tools for the care or management of excretion from people, such as the elderly and physically disabled people, suffering from excretory problems.

However, disposable diapers may be used not only by persons suffering from difficulty in urination on their own, but also by persons who can urinate on their own, such as physically disabled of mild incontinence and elderly persons who can walk. However, the physically disabled of mild incontinence and elderly persons who can walk have inhibitions about wearing a diaper, as they have the impression that the diaper is typically used by a person suffering from difficulty in urination by himself. This is because the conventional diapers have no urination opening or the like for drawing out the male sex organ from the viewpoint of preventing leakage, the opening being provided to typical male underwear. Thus, when urinating by himself, the wearer had to enter the cubicle, remove the diaper and urinate, for example. Such an act was very complicated, especially, for male wearers, which gave further inhibitions in wearing a diaper.

Japanese Unexamined Patent Application Publication No. 2005-080996 (refer hereafter to as Patent document 1) discloses an absorbent article that allows drawing-out/drawing-in of the male sex organ even during wearing of a diaper.

The absorbent article disclosed in Patent document 1 includes an inner sheet, an outer sheet, and an intermediate sheet interposed therebetween. The absorbent article further includes a paper-diaper main body that is divided into an abdomen part located on the side of a wearer's abdomen, a back part located on the side of a wearer's back, and a crotch part interposed therebetween during wearing and an absorbent core stuck to the crotch part of the paper-diaper main body. The inner surface of the abdomen part and the outer sheet are formed with perforations and an area for forming openings through which the male sex organ can be drawn out during wearing. By shifting the intermediate sheet, the openings of the inner surface of the abdomen part and the outer sheet communicate with each other to form a through hole in the abdomen part. With this, the absorbent article allows drawing-out/drawing-in of the male sex organ even when wearing the diaper.

### DISCLOSURE OF THE INVENTION

### Problems to be Solved by the Invention

With the absorbent article disclosed in Patent document 1, however, although the inner surface of the abdomen part and the inner sheet have openings for drawing out the male sex organ, the openings are configured as simple openings, and are thus difficult to enlarge in size, raising the problem of difficulty in drawing-out of the male sex organ. Moreover, when the wearer urinates, the absorbent article required various actions in order to draw out his male sex organ from the openings. For example, the wearer needed to disengage a fastening member provided to the intermediate sheet so as to shift the intermediate sheet. For wearers who can use only one hand, as a result of paralysis, etc., for example, the need of such an action made urination on their own difficult to carry out. Moreover, since the intermediate sheet was attached to the fastening member, specific sound could be generated when removing the intermediate sheet from the fastening member. With this, others could perceive wearing of the paper diaper during urination, which increases inhibitions associated with using the paper diaper.

The present invention is made in view of such problem, and aims to provide a disposable diaper that allows easy drawing-out of the male sex organ during urination.

### Means for Solving the Problems

In order to achieve the above-mentioned objective, the inventor has found that urination is facilitated by providing an opening in a predetermined location of the disposable diaper and forming a predetermined site thereof in a stretchable manner, and has reached completion of the present invention. Specifically, the present invention provides the disposable diaper of independent Claim 1. The dependent claims specify preferred but optional features.

In a first aspect, there is provided a disposable diaper, including: a chassis including at least a front body and a back body; a top sheet arranged on at least part of the chassis, the top sheet being liquid permeable; a back sheet arranged on the top sheet at one side in a thickness direction of the top sheet, the back sheet being liquid impermeable; and an absorbent core arranged between the top sheet and the back sheet, the absorbent core being liquid retainable, wherein the front body is formed with one of an openable part or an opening, the openable part and the opening each being formed by connecting two points positioned at a distance from each other across a substantial center line extending in a longitudinal direction of the disposable diaper and bisecting a width direction thereof, wherein the openable part allows the front body to be separated at a predetermined site, and the opening allows the front body to open at a predetermined site.

In a second aspect, the absorbent core is vertically long and is provided to at least the front body, wherein one of the openable part and the opening each are formed in the front body at a location away from a given outer edge of the absorbent core.

In a third aspect, the front body and the back body are joined and secured together at a predetermined location in advance or upon wearing the disposable diaper to provide a pants shape with a trunk opening and a pair of leg openings, in which an opening stretch area is constituted to connect a substantially central portion of the back body adjacent to the trunk opening and a predetermined location of the front body adjacent to at least one of the openable part or the opening along the outer perimeter of the pants shape, at least part of the opening stretch area being able to expand and contract.

In a fourth aspect, the opening stretch area in a stretched state is at least 1.1 times greater than that in a non-stretched state.

In a fifth aspect, the opening stretch area includes an elastic opening area arranged at a predetermined location on the side of the openable part or the opening in a leg direction of the pants shape.

In a sixth aspect, the opening stretch area includes an elastic back body area arranged in at least part of the back body adjacent to the trunk opening.

In a seventh aspect, the opening stretch area includes an elastic boundary area arranged in at least part of a boundary between the front body and the back body, the boundary including a joined position or an engaged position of the front body and the back body.

In an eighth aspect, the disposable diaper further includes an auxiliary sheet provided to the front body at a location including the openable part or the opening.

In a ninth aspect, the auxiliary sheet includes an elastic member at least in part.

In a tenth aspect, the auxiliary sheet is arranged with an absorbent core at least in part.

The present invention can provide a disposable diaper that allows easy drawing-out of the male sex organ during urination.

### BRIEF DESCRIPTION OF THE DRAWINGS

FIG. 1A is a front view of a disposable diaper of a first embodiment of the present invention;
FIG. 1B is a cross-sectional view of FIG. 1A taken along the line X-X;
FIG. 2 is an exploded perspective view of the disposable diaper in the first embodiment in the developed state;
FIG. 3A is a perspective view showing a worn state of pants-type disposable diaper;
FIG. 3B is a perspective view showing a worn state of developed-type disposable diaper;
FIG. 4A is a development view of the pants-type disposable diaper of the first embodiment;
FIG. 4B is a front view of the disposable diaper of FIG. 4A in a pants-shape;
FIG. 5A is a front view showing an opening of the disposable diaper in the first embodiment;
FIG. 5B is a front view showing another embodiment of the opening of the disposable diaper in the first embodiment;
FIG. 5C is a front view showing yet another embodiment of the opening of the disposable diaper in the first embodiment;
FIG. 6A is a front view showing a positional relationship between the opening and an absorbent core;
FIG. 6B is a development view showing a positional relationship between the opening and an absorbent core;
FIG. 7A is a perspective view showing a masking sheet as an auxiliary sheet;
FIG. 7B is a diagram showing another embodiment of the masking sheet;
FIG. 8A is a front view showing a joined position of the masking sheet;
FIG. 8B is a front view showing another joined position of the masking sheet;
FIG. 9A is a cross-sectional view showing the joined position of the masking sheet;
FIG. 9B is a cross-sectional view showing another joined position of the masking sheet;
FIG. 9C is a cross-sectional view showing another joined position of the masking sheet;
FIG. 9D is a cross-sectional view showing another joined position of the masking sheet;
FIG. 9E is a cross-sectional view showing another joined position of the masking sheet;
FIG. 10A is a front view showing a location of an elastic member;
FIG. 10B is a front view showing another location of the elastic member;
FIG. 11 is a perspective view showing a worn state of the disposable diaper in the first embodiment;
FIG. 12 is a perspective view showing the disposable diaper in the first embodiment, with the opening expanded;
FIG. 13A is a perspective view showing an elastic area of the disposable diaper of the first embodiment;
FIG. 13B is a perspective view showing a state in which the opening is expanded by extension of the elastic area thereof;
FIG. 14A is a perspective view showing an elastic area of the opening of the disposable diaper;
FIG. 14B is a perspective view showing a state in which the opening is expanded by extension of the elastic area thereof;
FIG. 15A is a perspective view showing an elastic back body area;
FIG. 15B is a perspective view showing a state in which the opening is expanded by extension of the elastic back body area;
FIG. 16A is a perspective view showing an elastic boundary area;
FIG. 16B is a perspective view showing a state in which the opening is expanded by extension of the elastic boundary area;
FIG. 17A is a developed view showing a disposable diaper in the second embodiment;
FIG. 17B is a developed view showing a positional relationship between the opening and absorbent core of the disposable diaper in the second embodiment;
FIG. 18A is a perspective view showing a disposable diaper in the second embodiment;
FIG. 18B is a sectional view taken along the line Y-Y in FIG. 18A;
FIG. 19 is a perspective view showing a worn state of the disposable diaper in the second embodiment;
FIG. 20 is a perspective view showing a state in which the opening of the disposable diaper in the second embodiment is expanded;
FIG. 21 is a perspective view showing another embodiment of the masking sheet;
FIG. 22 is a perspective view showing a worn state of a disposable diaper in the third embodiment;
FIG. 23 is a perspective view showing a state in which the opening of the disposable diaper in the third embodiment is expanded;
FIG. 24 is a developed view showing a disposable diaper in the fourth embodiment;
FIG. 25 is a perspective view showing a worn state of the disposable diaper in the fourth embodiment; and
FIG. 26 is a perspective view showing a state in which the opening of the disposable diaper in the fourth embodiment is expanded.

### PREFERRED MODE FOR CARRYING OUT THE INVENTION

Referring to the drawings, preferred embodiments of the present invention will be described hereafter. It is noted that the present invention and its technical scope are not limited thereto. For example, although an adult disposable diaper is described in the preferred embodiments, the present invention is not limited thereto, and can be applied to a disposable diaper for a child's toilet training, for example.

In the preferred embodiments, assuming that the side of the disposable diaper facing a wearer's body is a skin contact side, and the side opposite the skin contact side is a skin non-contact side.

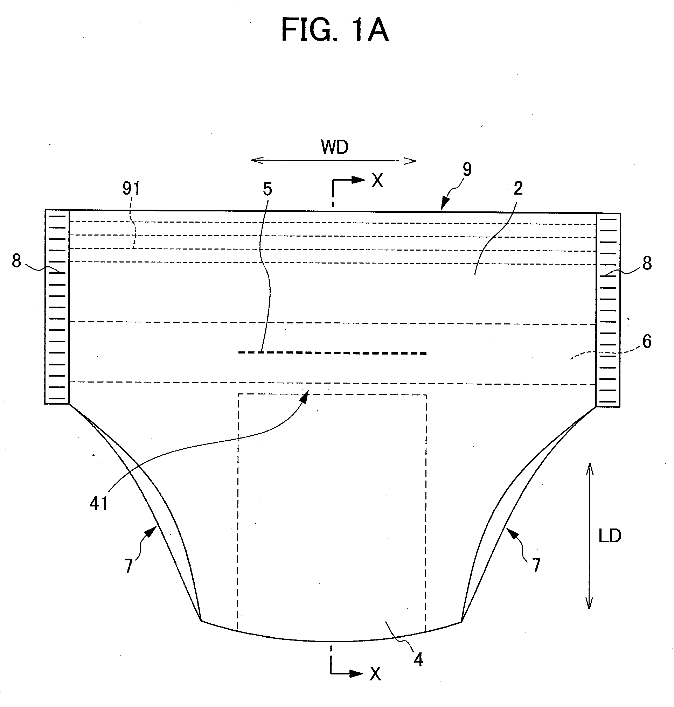

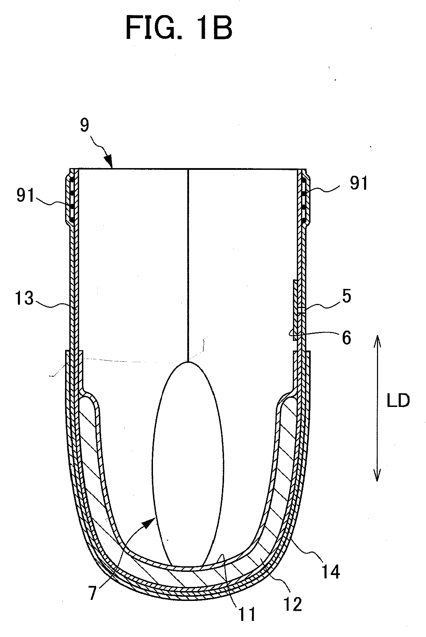

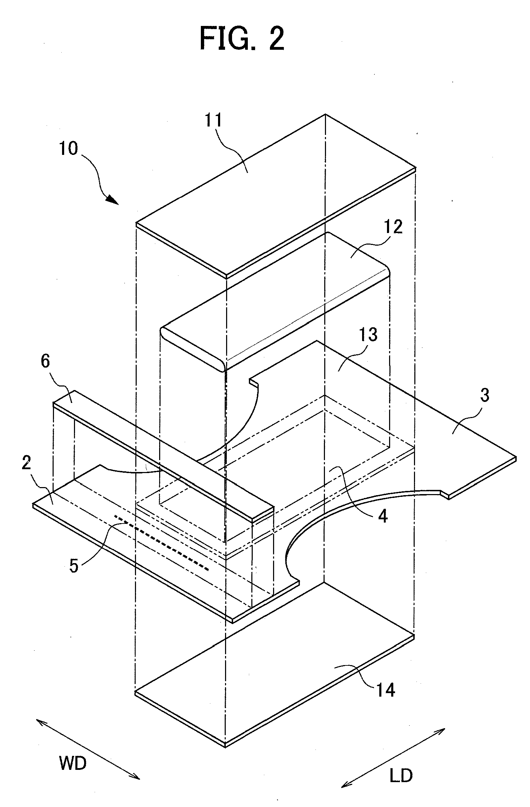

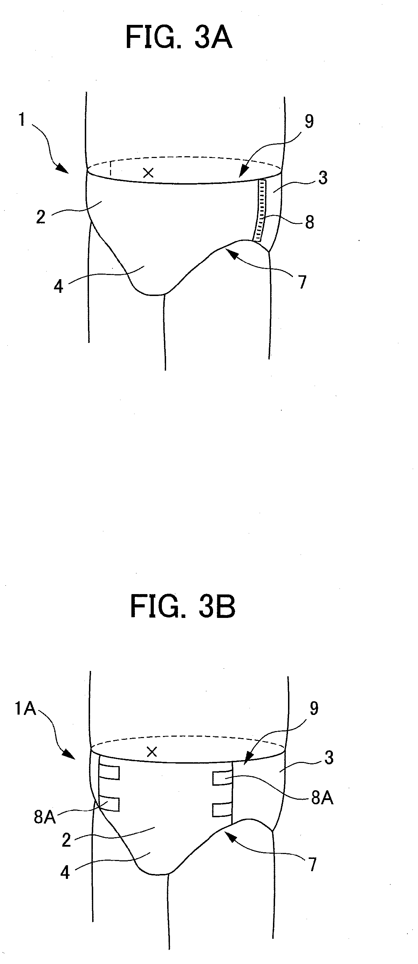

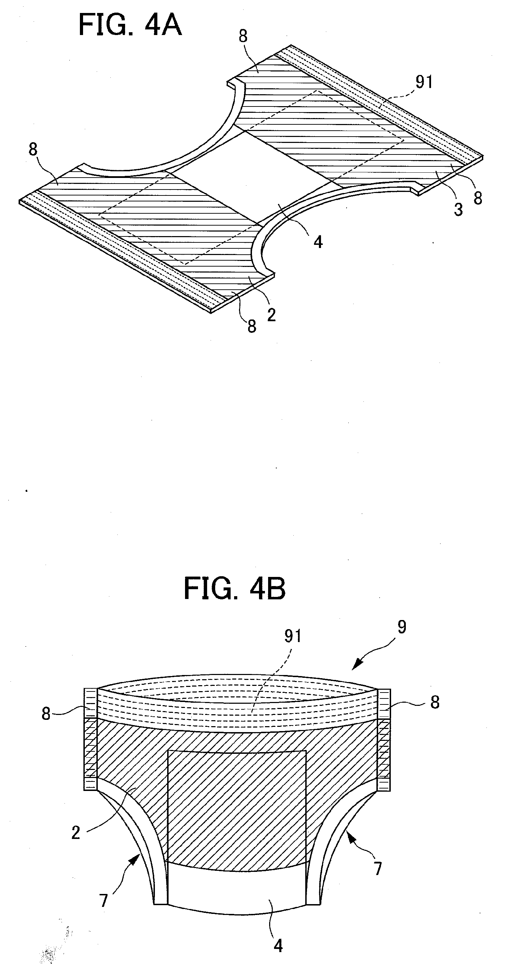

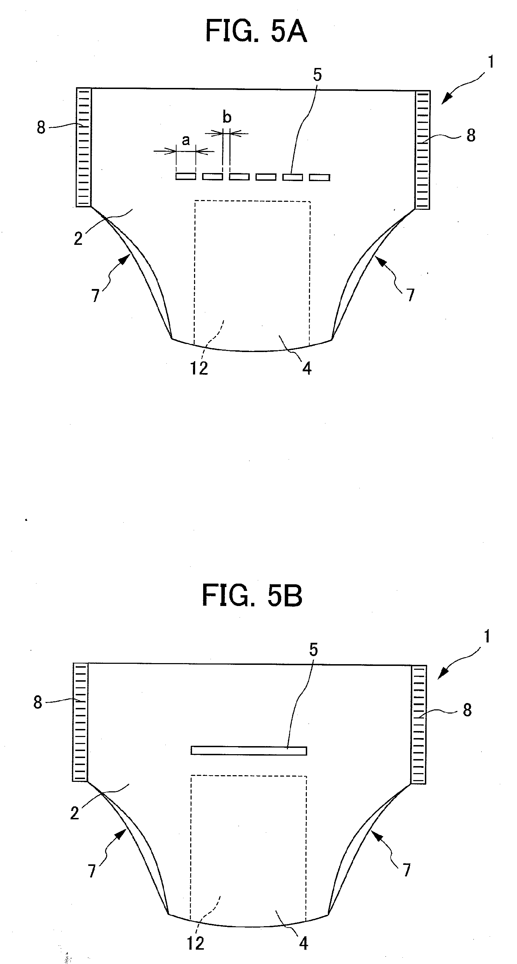

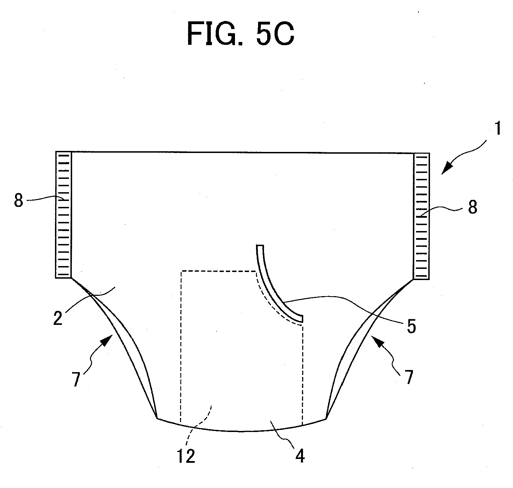

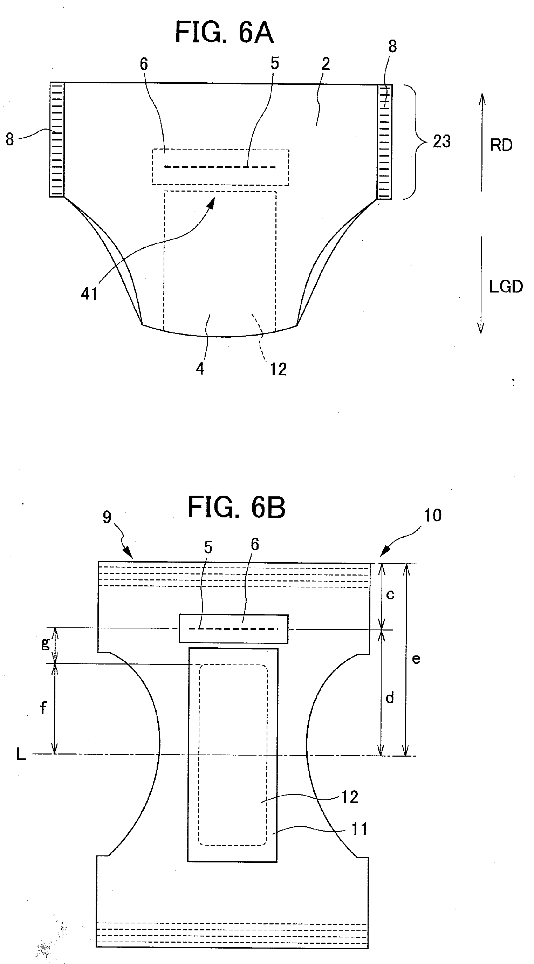

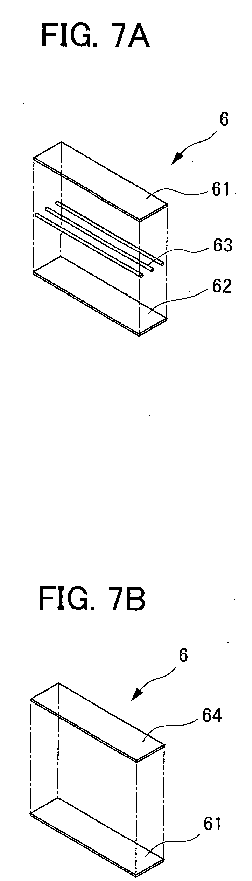

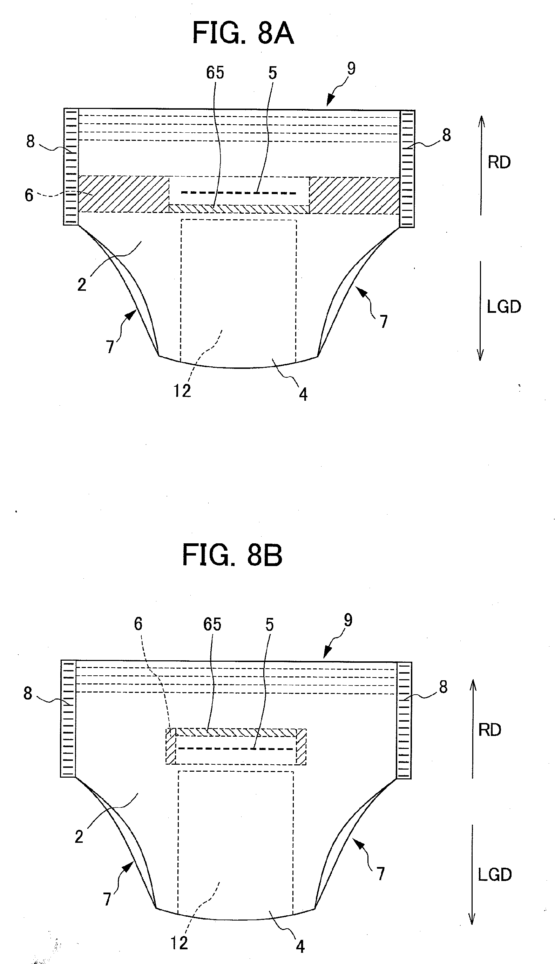

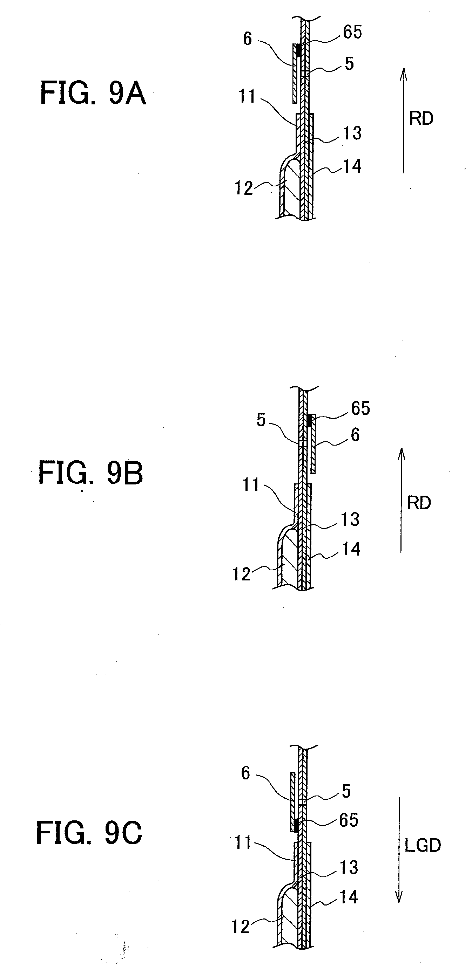

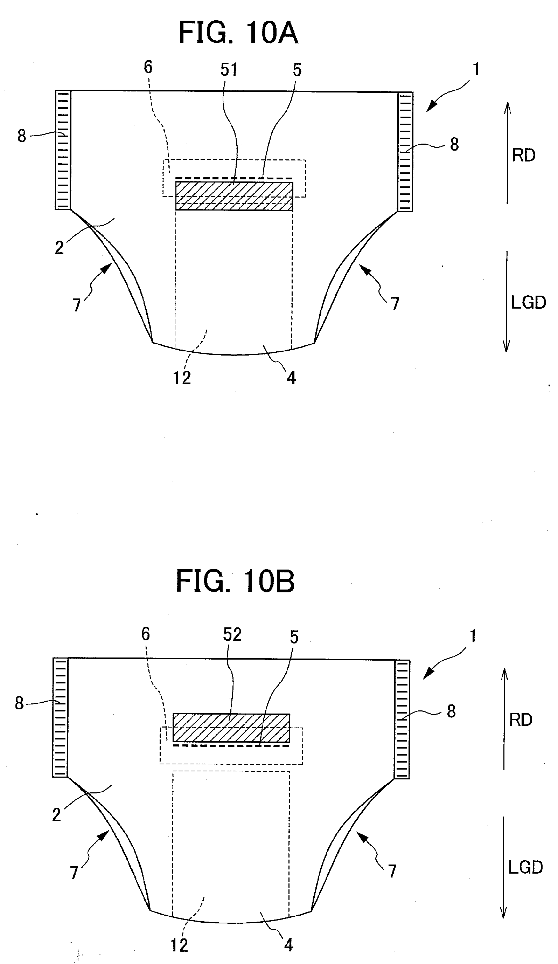

FIG. 1A is a front view showing a disposable diaper in the first embodiment of the present invention, and FIG. 1B is a sectional view taken along the line X-X in FIG. 1A. FIG. 2 is an exploded perspective view showing a disposable diaper in the first embodiment in the developed state. FIG. 3A is a perspective view showing a worn state of a pants-type disposable diaper, and FIG. 3B is a perspective view showing a worn state of a developed-type disposable diaper. FIG. 4A is a developed view showing a disposable diaper in the first embodiment, and FIG. 4B is a front view showing a disposable diaper of FIG. 4A as the pants-type. FIG. 5A is a front view showing an opening of the disposable diaper in the first embodiment, FIG. 5B is a front view showing another form of the opening, and FIG. 5C is a front view showing another form of the opening. FIG. 6A is a front view showing a positional relationship between the opening and absorbent core of the disposable diaper, and FIG. 6B is a developed view showing a positional relationship between the opening and the absorbent core. FIG. 7A is a perspective view showing a masking sheet serving as an auxiliary sheet, and FIG. 7B is a perspective view showing another form of the masking sheet. FIG. 8A is a front view showing a joined position of the masking sheet, and FIG. 8B is a front view showing another joined position of the masking sheet. FIGS. 9A to 9E are sectional views showing joined positions of the masking sheet. FIG. 10A is a front view showing a location of an elastic member, and FIG. 10B is a front view showing another location of the elastic member.

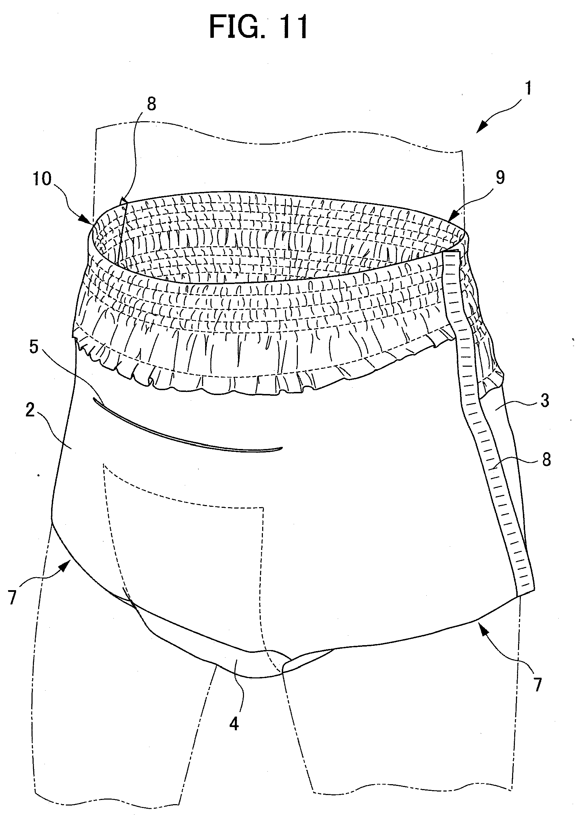

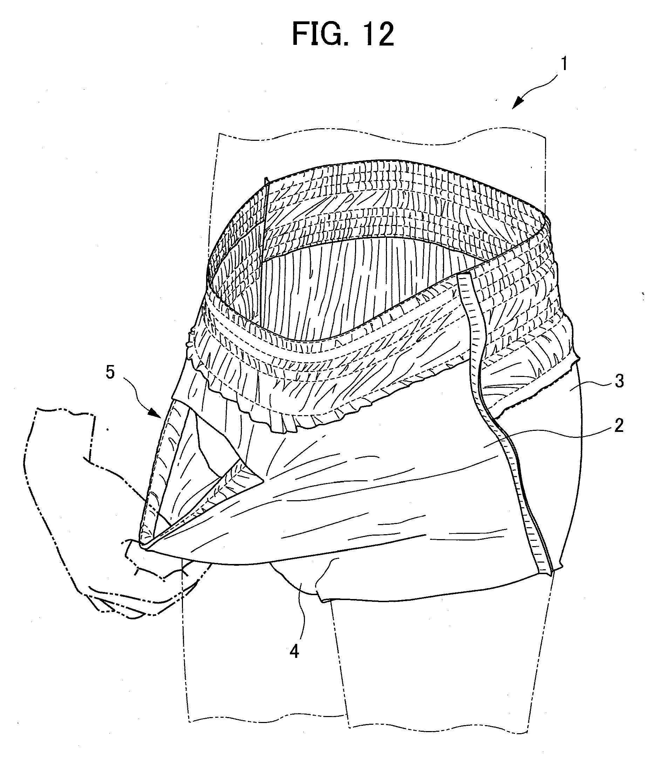

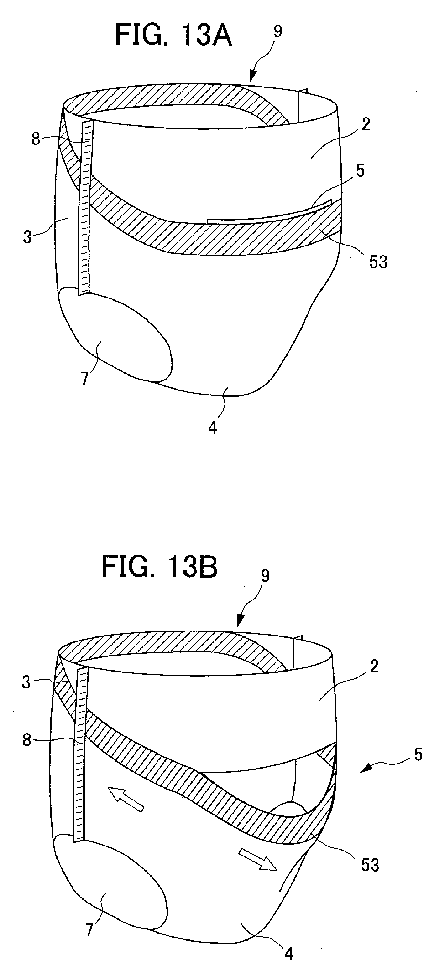

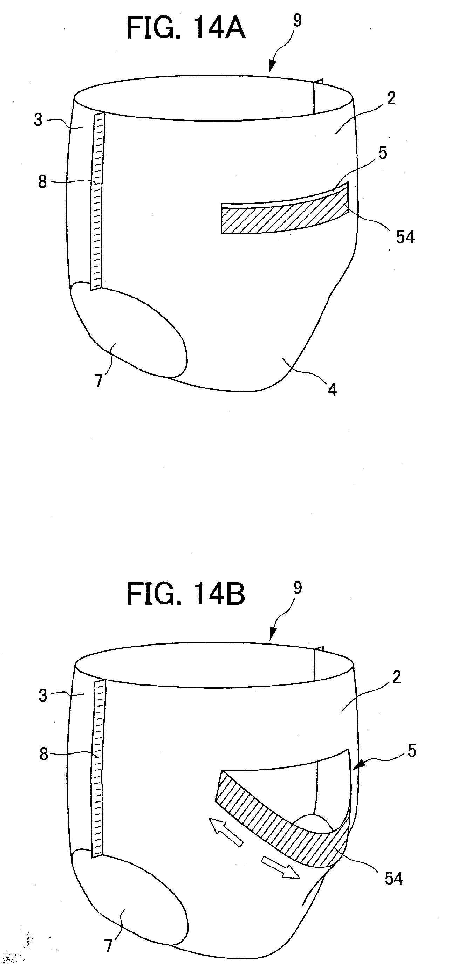

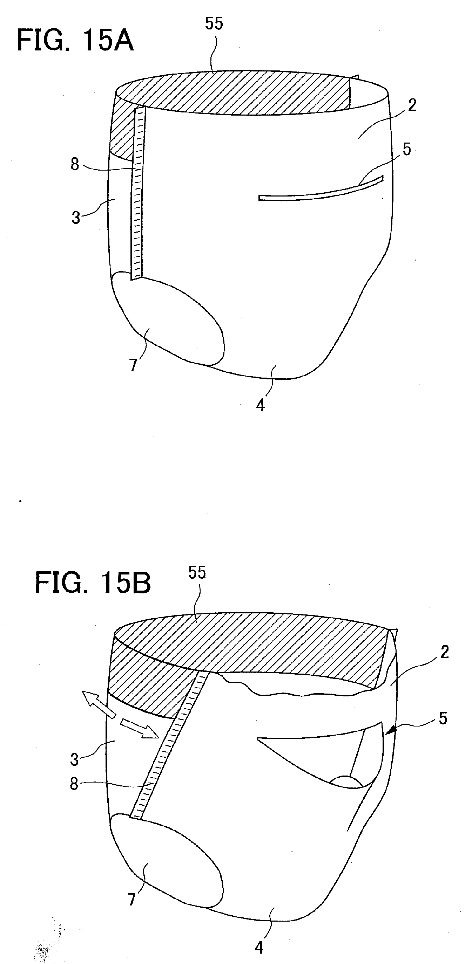

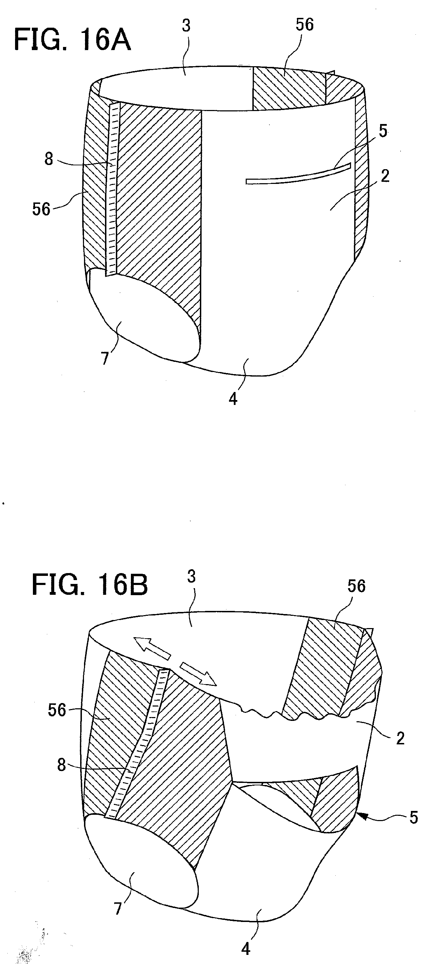

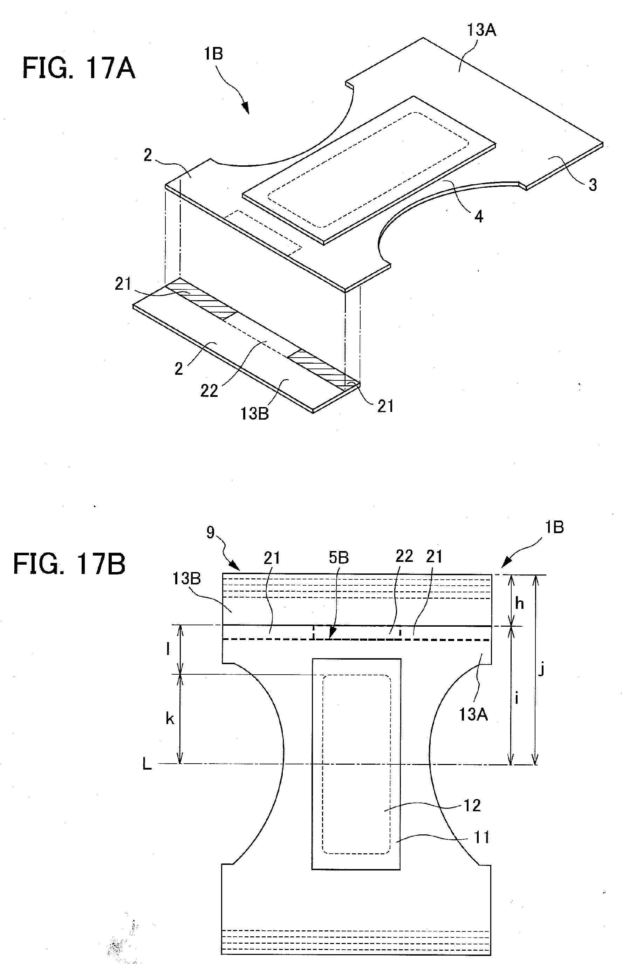

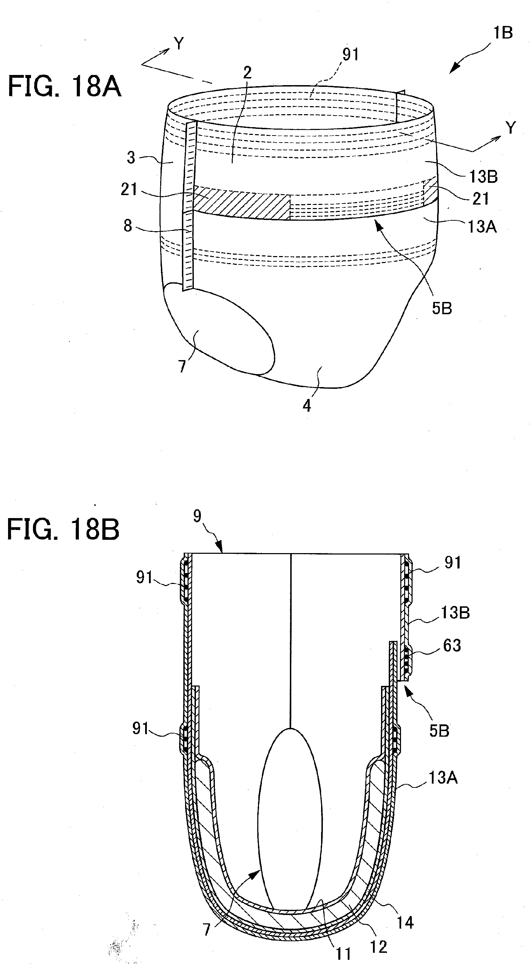

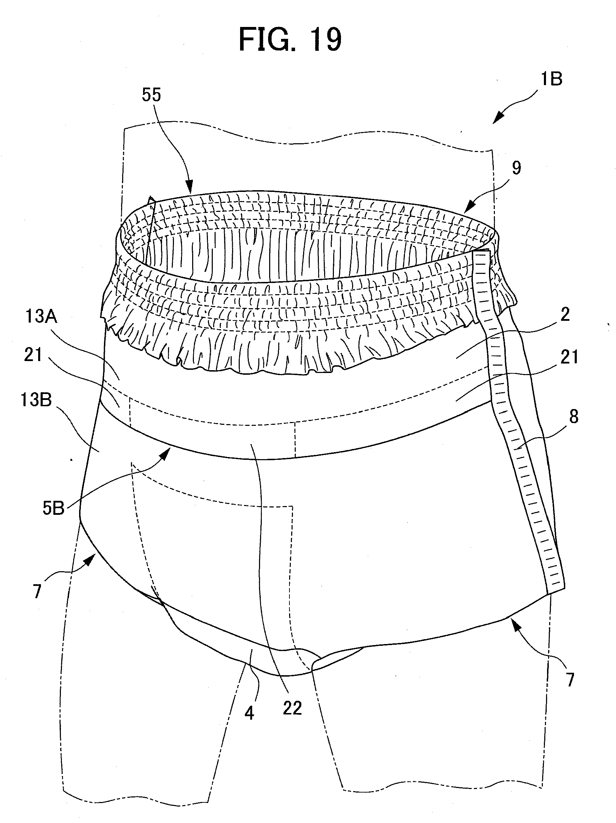

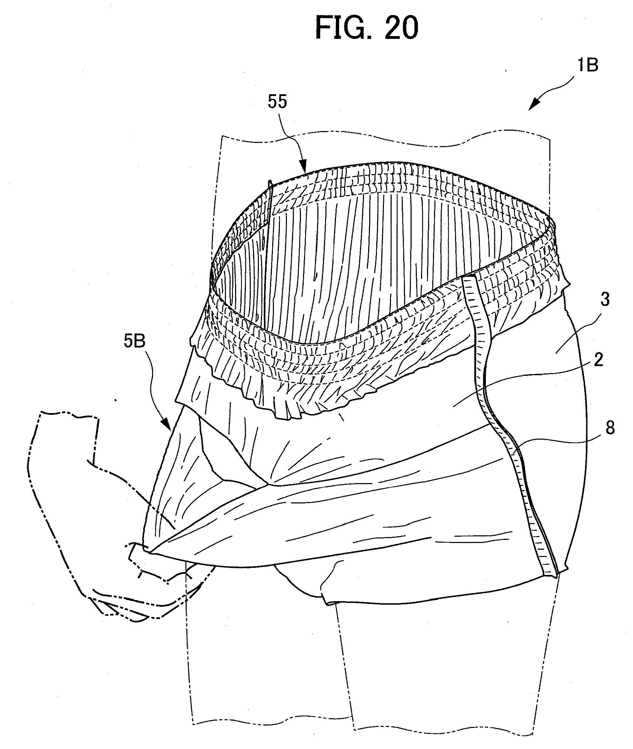

FIG. 11 is a perspective view showing a worn state of the disposable diaper in the first embodiment. FIG. 12 is a perspective view showing a state in which the opening of FIG. 11 is expanded. FIG. 13A is a perspective view showing an elastic area of the opening of the disposable diaper in the first embodiment, and FIG. 13B is a perspective view showing a state in which the opening is expanded by extension of the elastic area thereof. FIG. 14A is a perspective view showing an elastic area of the opening of the disposable diaper, and FIG. 14B is a perspective view showing a state in which the opening is expanded by extension of the elastic area thereof. FIG. 15A is a perspective view showing an elastic back body area, and FIG. 15B is a perspective view showing a state in which the opening is expanded by extension of the elastic back body area. FIG. 16A is a perspective view showing an elastic boundary area, and FIG. 16B is a perspective view showing a state in which the opening is expanded by extension of the elastic boundary area. FIG. 17A is a developed view showing a disposable diaper in the second embodiment, and FIG. 17B is a developed view showing a positional relationship between the opening and absorbent core of the disposable diaper in the second embodiment. FIG. 18A is a perspective view showing a disposable diaper in the second embodiment, and FIG. 18B is a sectional view taken along the line Y-Y in FIG. 18A. FIG. 19 is a perspective view showing a worn state of the disposable diaper in the second embodiment. FIG. 20 is a perspective view showing a state in which the opening of the disposable diaper in the second embodiment is expanded.

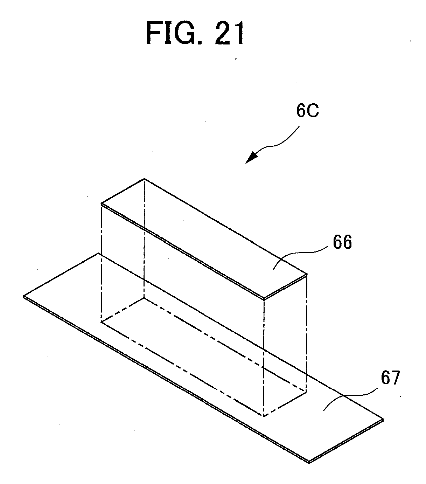

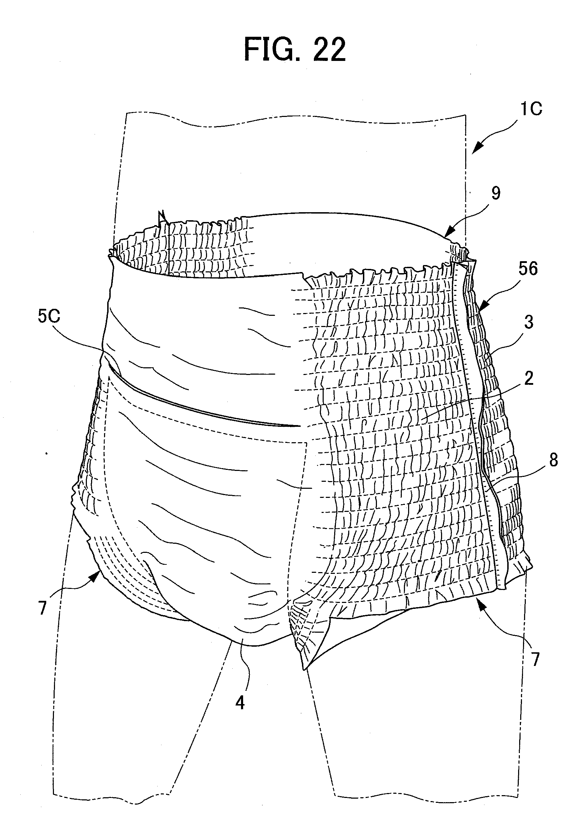

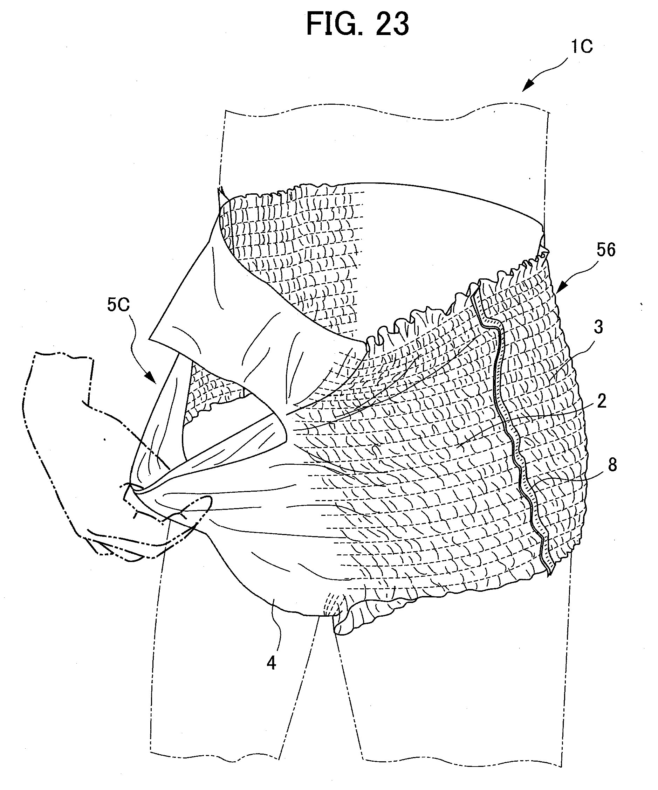

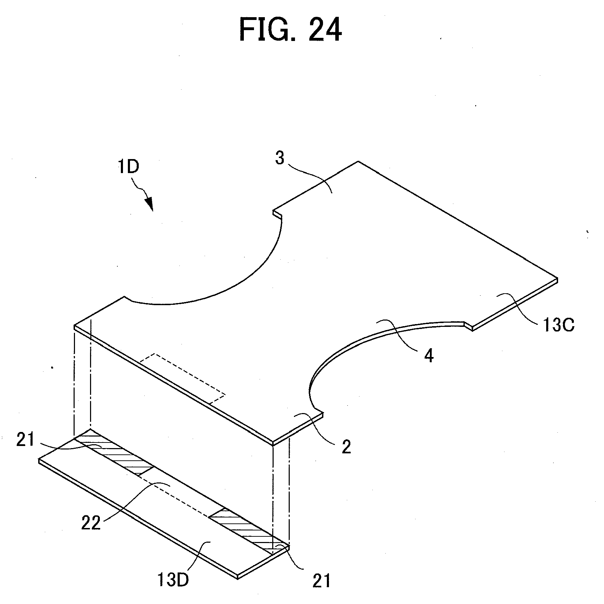

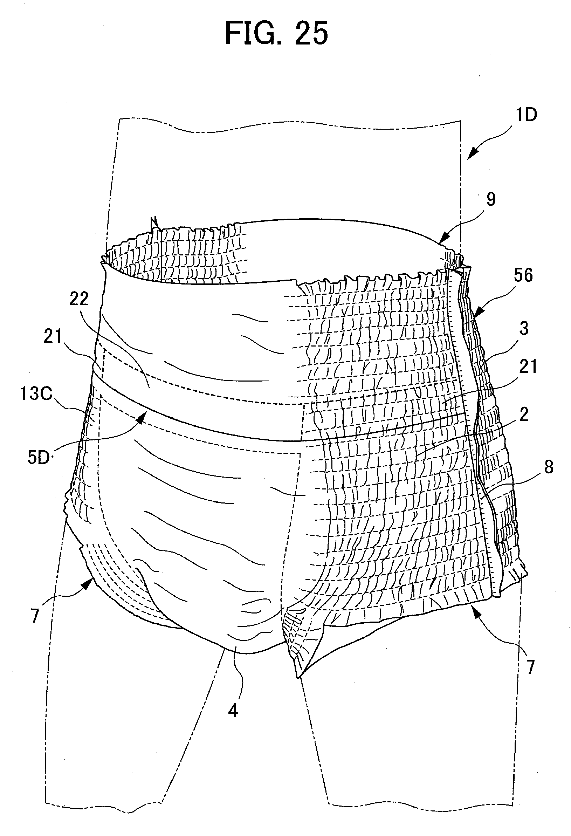

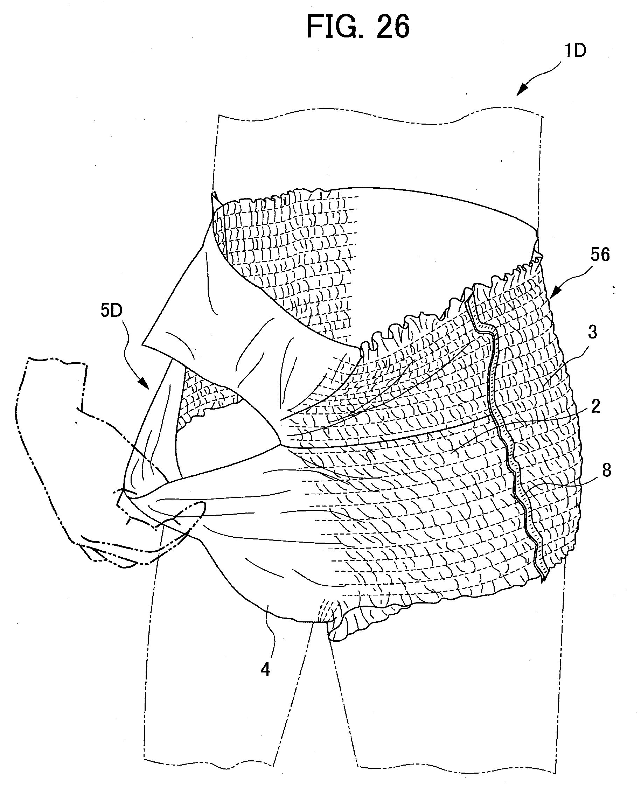

FIG. 21 is a perspective view showing another embodiment of the masking sheet. FIG. 22 is a perspective view showing a worn state of a disposable diaper in the third embodiment. FIG. 23 is a perspective view showing a state in which the opening of the disposable diaper in the third embodiment is expanded. FIG. 24 is a developed view showing a disposable diaper in the fourth embodiment. FIG. 25 is a perspective view showing a worn state of the disposable diaper in the fourth embodiment. FIG. 26 is a perspective view showing a state in which the opening of the disposable diaper in the fourth embodiment is expanded.

### 1. First embodiment

### 1.1. General configuration

The general configuration of the disposable diaper of the present invention will be described with regard to a disposable diaper 1 in the first embodiment. The disposable diaper 1 includes a main body 10 and an openable part 5 formed at a predetermined location of the main body 10. The openable part 5 is arranged to allow a wearer to draw out his male sex organ. Therefore, the disposable diaper 1 serves essentially as an article for males.

The openable part 5 is formed on a separation line that connects two points positioned at a distance across a substantial center line extending in the longitudinal direction LD or longitudinal direction of a front body 2 of the disposable diaper 1 and bisecting the width direction WD thereof. The openable part 5 is formed by intermittently arranging sections such as a slit or an opening on the separation line. When using the disposable diaper 1, first, a rise direction RD-side portion of the openable part 5 is pulled in the rise direction RD to separate portions other than the adjacent sections of the openable part 5. With this, the openable part 5 is expanded to be ready to use. The openable part 5 is covered with a masking sheet or auxiliary sheet 6.

### 1.2. Main body of the disposable diaper

Referring to FIGS. 1A to 4B, the main body 10 of the disposable diaper 1 includes a chassis 13 that forms the outer shape of the main body 10, a liquid-permeable top sheet 11 formed generally vertically long, a liquid-impermeable back sheet 14 formed generally vertically long, and a liquid-retainable absorbent core 12 formed generally vertically long. The chassis 13 is formed to provide a pants shape during wearing. The top sheet 11 is arranged on the skin contact side of the chassis 13 and constitutes a surface layer. The back sheet 14 is arranged on the skin non-contact side of the chassis 13, which is one side of the top sheet 11, and constitutes a back layer. The absorbent core 12 is arranged between the top sheet 11 and the chassis 13, i.e., between the top sheet 11 and the back sheet 14, and constitutes an absorption layer. It is noted that the wording "generally vertically long" includes a generally rectangular shape having longitudinal direction LD and width direction WD. Moreover, the wording includes a shape in which a portion of both sides in the longitudinal direction LD is dented to the center in the longitudinal direction LD or bulged in the direction opposite the center. Specifically, the absorbent core 12 includes an absorbent core that is different in length in the width direction WD in a portion in the longitudinal direction LD. Optionally, the absorbent core 12 may be arranged lengthwise either in the width direction WD or in the rise direction RD.

The absorbent core 12 may be arranged in the state of being covered with tissues (not shown) or a hydrophilic nonwoven fabric (not shown). When covered with a hydrophilic nonwoven fabric, the absorbent core 12 may be configured without application of the top sheet 11 or with only partial application thereof. This allows a reduction in manufacturing cost, for example. The back sheet 14 may include a back sheet having a nonwoven fabric or the like joined to the skin non-contact side. Joining a nonwoven fabric or the like to the skin non-contact side of the back sheet 14 is desirable in view of enhancement in wearer's feel during wearing, etc. When applying a liquid-impermeable film to the back sheet 14, disagreeable noises due to rubbing of the liquid-impermeable film, for example, can be prevented from occurring by joining a nonwoven fabric or the like to the liquid-impermeable film.

The chassis 13 includes a front body 2, a back body 3, and a crotch 4 arranged therebetween during wearing. The chassis 13 is formed to provide a pants shape by joining the front body 2 and the back body 3 at junctions 8 or predetermined locations of the front body 3 and the back body 3. Specifically, joining the front body 2 and the back body 3 at the junctions 8 provides a trunk opening 9 formed around a wearer's abdomen and a pair of leg openings 7 formed around wearer's legs in the worn state. It is noted that the predetermined location of the front body 2 and the back body 3 designates both ends of the front body 2 and the back body 3 in the direction (refer hereafter to as width direction WD) orthogonal to the rise direction RD in the worn state except the leg openings 7. The front and rear bodies 2 and 3 include, for example, front and rear bodies distinguished by the center line in the width direction WD, which bisects the disposable diaper 1 in the developed state in the longitudinal direction LD.

In this embodiment, a description is made about the pants-type disposable diaper 1 including trunk opening 9 and pair of leg openings 7 obtained by joining the front body 2 and the back body 3 at the junctions 8 as shown in FIG. 3A. Without being limited thereto, referring to FIG. 3B, the present invention can be applied to a developed-type disposable diaper 1A that can be worn by engaging the front body 2 and the back body 3 by engaging members 8A, etc. Optionally, the present invention can be applied to the disposable diaper 1A in which the front body 2 and the back body 3 are engaged by reengageable engaging members, such as a hook-and-loop fastener, which are used at the junctions 8 of the front body 2 and the back body 3 of the pants-type disposable diaper 1. This allows easy disengagement, development, and reengagement of the disposable diaper though the diaper is of the pants type.

The top sheet 11 is formed generally vertically long, and is arranged to extend from the front body 2 to the back body 3 of the chassis 13 along the crotch 4. Likewise, the absorbent core 12 is formed generally vertically long, and is arranged to extend from the front body 2 to the back body 3 of the chassis 13 along the crotch 4. The openable part 5 is formed in the front body 2 of the disposable-diaper main body 10 in a predetermined location of the chassis 13. For example, the openable part 5 is formed in the front body 2 in a location at a predetermined distance away from the absorbent core 12 so as to be generally parallel to an outer edge 41 or one end of the absorbent core 12 in the longitudinal direction LD.

Referring to FIGS. 4A and 4B, with the disposable-diaper main body 10, a plurality of filiform elastic members 91 are arranged at the circumference of the trunk opening 9. The elastic members 91 may be of the band type. The elastic members 91 may include an elastic sheet that can stretch, such as a stretchable nonwoven fabric (nonwoven fabric formed of mixed fibers of polyurethane and polypropylene or the like), a stretchable film or the like.

A filiform elastic member or an elastic sheet that can stretch, such as a stretchable nonwoven fabric or a stretchable film, may be provided to the front body 2 and the back body 3 of the disposable-diaper main body 2 in their entirety or in part. Moreover, the chassis 13 may include an elastic sheet that can stretch.

In the present invention, an anti-leakage wall or so-called leg gather assemblies (not shown) formed using an elastic member and an anti-leakage sheet may be arranged along both ends of the absorbent core 12 in the width direction WD. Specifically, the anti-leakage sheet may be arranged to extend in the width direction WD of the absorbent core 12 between the absorbent core 12 and the chassis 13 or the back sheets 14, wherein at least one elastic member is arranged at ends of the anti-leakage sheet in the width direction WD, and is fixed with a hotmelt adhesive or the like. The anti-leakage sheet may stay extending in the width direction WD of the absorbent core 12, or may be folded back to the center of the absorbent core 12 in the width direction WD to have the folded portion arranged on the skin contact side of the absorbent core 12.

With the leg gather assemblies, when extending from the absorbent core 12 in the width direction WD thereof, the distance between the absorbent core 12 and the elastic member of the leg gather assemblies is, preferably, in the range of more than 20 mm in the substantial center area of the absorbent core 12 in the longitudinal direction LD of the absorbent core 12. When the leg gather assemblies are folded back, the distance between the absorbent core 12 and the elastic member of the leg gather assemblies is, preferably, in the range of more than 20 mm in the state in which the anti-leakage sheet is extended in the width direction WD in the substantial center area.

With the main body 10 of the pants-type disposable diaper 1 before wearing, the length in the rise direction RD or maximum length in the rise direction RD from the crotch 4 to the trunk opening 9 is between 350 mm and 450 mm, for example. The maximum length between the junctions 8 in the direction (refer hereafter to as width direction WD) orthogonal to the rise direction RD in the developed state is between 425 mm and 900 mm, for example. It is noted that the developed state designates a state in which the front body 2 and the back body 3 are not joined as shown in FIG. 4A. The maximum length includes a length obtained by extending the contracted disposable-diaper main body 10 in the width direction WD and the rise direction RD or the leg direction LGD by an elastic force of filiform elastic members or substrate sheets as will be described later.

With the disposable diaper 1 for children, the length of the main body 10 of the pants-type disposable-diaper 1 in the rise direction RD before wearing is between 200 mm and 300 mm, for example. The maximum length between the junctions 8 in the width direction WD of the disposable-diaper main body 10 in the developed state is between 300 mm and 450 mm, for example.

In this embodiment, the back sheet 14 is arranged on the wearer's skin non-contact side. Without being limited thereto, the back sheet 14 may be arranged between the absorbent core 12 and the chassis 13 or between sheets when the chassis 13 includes a plurality of sheets.

### 1.3. Openable part

Referring to FIG. 5A, the openable part 5 is obtained by, for example, intermittently (at intervals b) forming sections, such as slits or openings (like perforations, for example). By breaking the portions interposed between the adjacent sections, the openable part 5 can provide an opening of a predetermined size. The maximum length of the openable part 5 in the developed state is more than 100 mm and in the range of less than 60% of the maximum length of the disposable-diaper main body 10 in the width direction WD in the developed state, for example. Preferably, the maximum length is more than 120 mm and in the range of less than 40% of the length of the disposable-diaper main body 10 in the width direction WD. This is because the openable part 5 requires a length of more than 100 mm to allow the wearer to draw out his male sex organ. When the length of the openable part 5 exceeds the range of 60% of the length of the disposable-diaper main body 10 in the width direction WD, the disposable diaper 1 is difficult to pull up during wearing, leading to poor ease of use.

Specifically, when the length of the disposable diaper 1 in the width direction WD is 550 mm, the length of the openable part 5 is 200 mm.

With the pitch of the sections a as formed intermittently, the sections a in the range from 1.0 mm to 20.0 mm can be continuously formed at intervals b in the range from 0.5 mm to 5.0 mm, for example. Preferably, the range of the sections a is from 2.0 mm to 8.0 mm, and the range of interval b is from 1.0 mm to 3.0 mm. Preferably, the length of the sections a is equal to or greater than the length of the intervals b.

A cleavage (not shown) of 25 mm to 60 mm may be arranged substantially in the center of the openable part 5. It is noted that the cleavage designates a cut formed in a portion of the chassis 13 corresponding to the openable part 5. Thus, arranging the cleavage substantially in the center of the openable part 5 allows the wearer to insert his fingers, etc. into the cleavage so as to easily expand therefrom the openable part 5, for example. When stiffening members as will be described later are arranged at both ends of the openable part 5, the cleavage is arranged, preferably, in advance in a portion of each stiffening member located in the openable part 5. This is because even if a plurality of sections are to be intermittently formed in a continuous manner and are arranged in the portion of the stiffening member located in the openable part 5, portions between the sections a will become difficult to separate due to presence of the stiffening member.

Specifically, the length of the cleavage is 40 mm. The length of the cleavage arranged in the stiffening member area in the openable part 5 is 30 mm. With the openable part 5 having a 200 mm length, for example, the length of the cleavage arranged in the stiffening member at each end of the openable part 5 is 30 mm, the length of the cleavage arranged substantially in the center of the openable part 5 is 40 mm, the length of the sections a located between the cleavage at each end of the openable part 5 and the cleavage substantially in the center thereof is 4.0 mm, and the length of the interval b is 1.0 mm.

In this embodiment, the openable part 5 is obtained by intermittently forming the sections, such as slits or openings, in a continuous manner. Without being limited thereto, referring to FIG. 5B, the openable part 5 may be formed by arranging a separation-line part wherein the thickness and thus strength of the corresponding portion of the chassis 13 is reduced with respect to other portions thereof. In this embodiment, the openable part 5 is formed like a straight line. Without being limited thereto, the openable part may be formed like a curve and a combination of a straight line and a curve, for example. Optionally, referring to FIG. 5C, by cutting a portion of the absorbent core 12, the openable part 5 may be formed with a curve or straight line with respect to the cut. Optionally, by arranging in a portion of the absorbent core 12 an area for openable part 5, the openable part 5 may be arranged inside the absorbent core 12.

When the openable part 5 is obtained by intermittently forming the sections a such as slits or openings or arranging the separation-line part, the stiffening members (not shown) are joined, preferably, to both ends of the openable part 5 of the chassis 13 so as not to break both ends of the openable part 5. When the chassis 13 includes a plurality of components, the stiffening members may be interposed between the components and at the sites corresponding to both ends of the openable part 5.

The stiffening members may be formed of a nonwoven fabric, a film or the like having more than 5N tear strength (measuring method: A-1 (single tongue method), JIS L 1906) both in the width direction WD and in the longitudinal direction LD, for example. The size of the stiffening members is 60 mm long (rise direction RD) and 40 mm wide (width direction WD), for example. The stiffening members may be joined to both ends of the openable part 5 using hotmelt adhesion, heat-sealing, ultrasonic sealing, etc., for example. When the chassis 13 constituting the front body 2 includes a plurality of components, the stiffening members may be arranged between the components, which are then joined or fixed from both surfaces.

The openable part 5 may be formed, at both ends thereof, with a shape of a smooth curve such as a circle or an ellipse, for example. Specifically, holes having shapes of a smooth curve such as a circle or an ellipse may be arranged at both ends of the openable part 5. Thus, arranging holes of a predetermined shape at both ends of the openable part 5 allows increasing the anti-tear strength of the openable part 5 at both ends when expanding the openable part 5. Moreover, with the openable part 5 having other configuration than the configuration of intermittently forming the sections such as slits or openings or the configuration of forming the separation-line part, arranging holes of a predetermined shape at both ends of the openable part 5 allows dispersion of forces applied to both ends of the openable part 5 when expanding the openable part 5, leading to prevention of breakage of both ends of the openable part 5.

Moreover, cut lines may be arranged at both end of the openable part 5 to extend in the direction different from the direction of the slit or the like or the separation-line part of the openable part 5. The cut lines arranged at both ends may be any of an arc, a curve, or a straight line. Thus, arranging the cut lines at both ends of the openable part 5 to extend in predetermined directions allows increasing the anti-tear strength of the openable part 5 at both ends when expanding the openable part 5.

In this embodiment, the openable part 5 is configured to be like a straight line by the sections such as slits or openings that are formed intermittently in a continuous manner. Without being limited thereto, the openable part 5 may be configured like a curve, a straight line or a combination thereof, for example, wherein the curve or the straight line connects two points positioned at a distance across the substantial center line extending in the longitudinal direction LD of the front body 2 of the disposable diaper 1 and bisecting the width direction WD thereof. Specifically, the openable part 5 may be formed like a waveshape, a mountain shape, etc. Moreover, the openable part 5 may be configured so that part or all of the openable part 5 can be cut off. For example, the openable part 5 may be formed in the shape of a circle, an ellipse, a polygon or the like, wherein part or all of the portion can be cut off.

For the purpose of easy recognition of the location of the openable part 5, the openable part 5 may be colored in the rise direction RD side or leg-direction side of the openable part 5 or provide the irregularities by embossing, etc., for example. Thus, the openable part 5 may be configured to provide a sign for facilitating recognition of the location of the openable part 5 visually or tactually. Providing such visual or tactual signs allows easy recognition of the location of the openable part 5 when the wearer urinates by himself, for example, leading to easier urination. Moreover, when assisting urination of a bedridden patient, etc., for example, a caregiver can easily recognize the location of the openable part 5 of the disposable diaper 1 worn by the patient, etc., leading to the facilitation of providing assistance to in care patients.

An opening auxiliary such as a handle may be provided to the openable part 5. Providing the opening auxiliary allows the wearer to easily separate the openable part 5 using the opening auxiliary, for example. Moreover, this allows easy enlargement of the openable part 5 when assisting in care bedridden patients urinate, for example.

### 1.4. Location of the openable part

Referring to FIG. 6A, the openable part 5 is located in the range of a trunk periphery 23 of the front body 2. Preferably, the openable part 5 is arranged at a location of the front body 2 at a predetermined distance away from one side or a given outer edge 41 of the generally vertically long absorbent core 12, which extends in the width direction WD. It is noted that "location a predetermined distance away" includes a location away in the rise direction RD and a location away in the leg direction LGD, for example. Therefore, the openable part 5 may be arranged in part of the absorbent core 12 on condition that the openable part 5 is located in the range of the trunk periphery 23. In this case, the absorbent core 12 is formed, preferably, with its periphery around the openable part 5 being sealed so as not to leak a material of the absorbent core 12.

With the location of the openable part 5, referring to FIG. 6B, assuming that the distance from the end of the trunk opening 9 to the lower end of the openable part 5 is c, the distance from the lower end of the openable part 5 to a longitudinal direction center L in the developed state is d, and the distance from the end of the trunk opening 9 to the longitudinal direction center L in the developed state is e, a value of d/c is, preferably, in the range from 0.65 to 8.0, for example.

Specifically, the distance c from the end of the trunk opening 9 to the lower end of the openable part 5 is, preferably, in the range from 50 mm to 270 mm. The distance d from the lower end of the openable part 5 to the longitudinal direction center L in the developed state is, preferably, in the range from 180 mm to 400 mm. The distance e from the end of the trunk opening 9 to the longitudinal direction center L in the developed state is, preferably, in the range from 350 mm to 450 mm.

Moreover, the openable part 5 is arranged, preferably, on the rise direction RD side of the absorbent core 12. Specifically, assuming that the distance from the longitudinal direction end of the absorbent core 12 in the front body 2 to the longitudinal direction center L in the developed state is f, and the distance from the lower end of the openable part 5 to the longitudinal direction end of the absorbent core 12 in the front body 2 is g, a value of d/f is, preferably, in the range from 1.0 to 2.3. Specifically, the distance f from the longitudinal direction end of the absorbent core 12 in the front body 2 to the longitudinal direction center L in the developed state is, preferably, in the range from 175 mm to 395 mm. The distance g from the lower end of the openable part 5 to the longitudinal direction end of the absorbent core 12 in the front body 2 is, preferably, in the range from 5 mm to 225 mm.

With a disposable diaper smaller than the disposable diaper 1 in this embodiment, i.e., disposable diaper for children so called, the distance c from the end of the trunk opening 9 to the lower end of the openable part 5 is, preferably, in the range from 35 mm to 180 mm, for example. The distance d from the lower end of the openable part 5 to the longitudinal direction center L in the developed state is, preferably, in the range from 120 mm to 265 mm. The distance e from the end of the trunk opening 9 to the longitudinal direction center L in the developed state is, preferably, in the range from 200 mm to 300 mm.

With the absorbent core 12 for children, the distance f from the longitudinal direction end of the absorbent core 12 in the front body 2 to the longitudinal direction center L in the developed state is, preferably, in the range from 115 mm to 260 mm. The distance g from the lower end of the openable part 5 to the longitudinal direction end of the absorbent core 12 in the front body 2 is, preferably, in the range from 5 mm to 150 mm.

### 1.5. Masking sheet

Referring to FIG. 7A, the masking sheet 6 is formed generally rectangularly. The masking sheet 6 is joined to the skin contact side or skin non-contact side of the chassis 13 in such a manner as to cover the openable part 5. The length of the masking sheet 6 in the longitudinal direction LD is greater than the length of the openable part 5 in the width direction WD. Specifically, the masking sheet 6 only needs to have a length that allows covering of the openable part 5 in the entire width direction WD.

The masking sheet 6 is formed by holding a plurality of filiform elastic members 63 in the extended state by a pair of band-like substrate sheets 61 and 62, bonding the substrate sheets 61 and 62 together, and releasing the tension of the elastic members 63. With this, the masking sheet 6 provides a plurality of slacks or so-called gathers. The masking sheet 6 can be extended by an extendable part of the gathers. In other words, the gathers will disappear by extending the masking sheet 6. Thus, the masking sheet 6 can make contact with a wearer's abdomen by an elastic force of the elastic members 63 during wearing, and can follow the expanded state of the openable part 5 and extend by a part of the gathers when the wearer expands the openable part 5, for example.

Referring to FIG. 7B, the masking sheet 6 may be formed by bonding a band-like elastic member 64 and the substrate sheet 61 together. Alternatively, the masking sheet 6 may be formed by holding the band-like elastic member 64 by the pair of substrate sheets 61 and 62 and bonding the substrate sheets 61 and 62 together. Optionally, the masking sheet 6 may be formed by folding the substrate sheet 61 and holding the elastic members 63 and 64 therebetween. The band-like elastic member may be a substrate sheet such as an elastic nonwoven fabric, which can be used as the masking sheet 6.

The masking sheet 6 is joined to the chassis 13 at a junction 65 in such a manner as to cover all the openable part 5 formed in the front body 2. With the junction 65 of the masking sheet 6 and chassis 13, referring to FIG. 8A, the masking sheet 6 may be joined to the chassis 13 at a portion of the openable part 5 in the leg direction LGD or at both ends of the masking sheet 6 in the longitudinal direction LD, for example. Alternatively, referring to FIG. 8B, the masking sheet 6 may be joined to the chassis 13 at a portion of the openable part 5 in the rise direction RD or at both ends of the masking sheet 6 in the longitudinal direction LD. The junction 65 may be a continuous or discontinuous junction made with respect to the chassis 13.

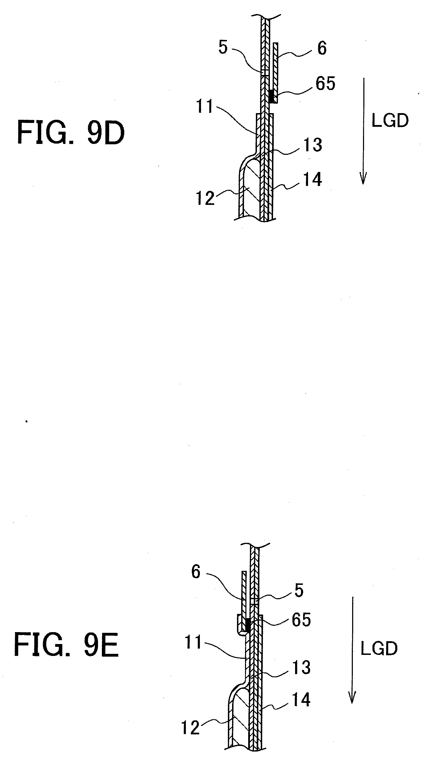

Referring to FIGS. 9A to 9D, the masking sheet 6 may be joined to the skin contact side of the chassis 13 via the rise direction RD of the openable part 5 as shown in FIG. 9A, or to the skin non-contact side of the chassis 13 via the rise direction RD of the openable part 5 as shown in FIG. 9B. Optionally, the masking sheet 6 may be joined to the skin contact side of the chassis 13 via the leg direction LGD of the openable part 5 as shown in FIG. 9C, or to the skin non-contact side of the chassis 13 via the leg direction LGD of the openable part 5 as shown in FIG. 9D. Referring to FIG. 9E, when the masking sheet 6 is joined to the skin contact side of the chassis 13 in the leg direction LGD of the openable part 5, the masking sheet 6 may be formed in such a manner as to cover the junction 65 with the top sheet 11.

Thus, the masking sheet 6 includes elastic members 63 in its entirety or in part. For example, when the masking sheet 6 is arranged on the skin non-contact side of the openable part 5, the masking sheet 6 allows the chassis 13 in the openable part 5 to make contact with a wearer's skin, etc. and to prevent the wearer's skin, etc. from being exposed. When the masking sheet 6 is arranged on the skin contact side of the openable part 5, the masking sheet 6 allows the chassis 13 in the openable part 5 to be drawn to the wearer's skin and to prevent the wearer's skin, etc. from being exposed.

The masking sheet 6 may be arranged with an absorbent core in its entirety or in part. With this, during urination, even if urine is discharged before drawing out the male sex organ, for example, discharged urine can be absorbed by the masking sheet 6.

### 1.6. Elastic member adjacent to the openable part

Referring to FIG. 10A, when the masking sheet 6 is joined to the skin contact side of the chassis 13 via the rise direction RD of the openable part 5 (refer to FIG. 9 A), an elastic member is arranged, preferably, in at least part of a crotch area 51 adjacent to the openable part 5. The elastic member may be either filiform or like a sheet.

Referring to FIG. 10B, when the masking sheet 6 is joined to the skin contact side of the chassis 13 in the leg direction LGD of the openable part 5 (refer to FIG. 9C), the elastic member is arranged, preferably, in at least part of a rise area 52 adjacent to the openable part 5. The elastic member may be either filiform or like a sheet.

Thus, by joining to the chassis 13 the masking sheet 6 having an elastic member arranged, the openable part 5 can be opened and closed as required without exposing a wearer's skin.

### 1.7. Usage mode

A description will be made about the usage mode of the disposable diaper 1 in the first embodiment of the present invention.

Referring to FIG. 11, in this embodiment, the disposable diaper 1 is of the pants type, which can be worn and used like typical pants by a wearer. During urination, the wearer pulls part of a rise direction RD part of the front body 2 adjacent to the openable part 5 having sections such as slits and openings formed intermittently, or part of the front body 2 at the trunk opening 9, cleaving and expanding the openable part 5. Subsequently, referring to FIG. 12, the wearer holds or catches a substantial center of the opening of the openable part 5 with his fingers, etc., and pulls it in the leg direction LGD. With this, the opening of the openable part 5 is extended in the leg direction LGD to allow securing of the opening of a predetermined size. Specifically, referring to FIGS. 13A and 13B, when the wearer holds or catches a substantial center of the opening of openable part 5 with his fingers, etc., and pulls it in the leg direction LGD, at least part of an opening stretch area 53 (refer to FIG. 13A) is extended by an elastic force, the area 53 being an area connecting a substantially central portion of the back body 3 adjacent to the trunk opening 9 and a substantially central portion of the front body 2 adjacent to the openable part 5 along the outer perimeter of the pants shape. Then, the opening of the openable part 5 is extended in the leg direction LGD to allow securing of the opening of a predetermined size (refer to FIG. 13B).

With a stretch part, referring to FIGS. 14A and 14B, using a stretchable elastic member, an elastic opening area 54 may be arranged on the leg-direction side of the openable part 5 in the open direction to serve as the opening stretch area 53, for example. Alternatively, referring to FIGS. 15A and 15B, using a stretchable elastic member, an elastic back body area 55 may be arranged on the perimeter of the back body 3 adjacent to the trunk opening 9 to serve as the opening stretch area 53, for example. Optionally, referring to FIGS. 16A and 16B, using a stretchable elastic member, an elastic boundary area 56 may be arranged in all or part of a boundary area including a joined or engaged position between the front body 2 and the back body 3 to serve as the opening stretch area 53, for example. Optionally, a combination of the elastic opening area 54, elastic back body area 55, and elastic boundary area 56 may be arranged to serve as the opening stretch area 53.

When the openable part 5 does not provide an opening before use due to the formation of the sections, such as slits and openings that are formed intermittently in a continuous manner, etc., the openable part 5 should be opened in advance by pulling part of the front body 2 in the leg direction LGD or in the rise direction RD.

Thus, the wearer can secure the opening of a predetermined size with a single hand, leading to the easy drawing-out of his male sex organ. With this, the wearer can easily urinate without removing the diaper 1. Moreover, the openable part 5 has the open direction conforming to the leg direction LGD, and the opening shape extending in the width direction WD of the disposable diaper 1, leading to the easy expansion of the openable part 5 by either a right-handed wearer or a left-handed wearer. Moreover, since the masking sheet 6, which can be stretched, is joined to the skin contact side of the openable part 5, the chassis 13 can make contact with a wearer's abdomen during ordinary wearing and after urination, leading to prevention of the skin from being unnecessarily exposed.

### 2. Other embodiments

Referring to FIGS. 17A to 26, the second to fourth embodiments of the present invention will be described. The second embodiment depicts an implementation in which an opening 5B is formed by superimposing chassis 13A and 13B one upon another at a predetermined location of the front body 2. The third embodiment depicts an implementation in which an elastic member is arranged at the boundary between the front body 2 and the back body 3 to reduce stress acting on parts of an openable part 5C in the rise direction RD and in the leg direction LGD as compared with stress acting on other parts. The fourth embodiment depicts an implementation in which an opening 5D is formed by superimposing chassis 13C and 13D one upon another at a predetermined location of the front body 2, wherein an elastic member is arranged at the boundary between the front body 2 and the back body 3 to reduce stress acting on parts of the opening 5D in the rise direction RD and in the leg direction LGD as compared with stress acting on other parts.

In the following embodiments, omission of the description means that the description has been made in connection with the first embodiment, and like references designate like parts in the first embodiment.

### 2.1. Second embodiment

Referring to FIGS. 17A to 20, a disposable diaper 1B in the second embodiment of the present invention will be described. As seen from FIGS. 17A to 20, the disposable diaper 1B in the second embodiment differs from the disposable diaper 1 in the first embodiment in the configuration of forming the opening 5B. Specifically, referring to FIGS. 17A and 17B, a rectangular chassis 13B is superimposed upon a chassis 13A by a predetermined amount at a predetermined location of the front body 2 to provide a superimposed portion, and the chassis 13B and 13A are joined together at a pair of junctions 21 or both sides of the superimposed portion, forming the front body 2. The superimposed portion comes into a state where it is able to be opened, by arranging a non-joined part 22 substantially in the center of the front body 2. Thus, in the second embodiment, the opening 5B is formed in the disposable diaper 1B.

The maximum length of the opening 5B in the developed state is more than 100 mm and in the range of less than 60% of the maximum length of the disposable-diaper main body 10 in the width direction WD in the developed state, for example. Preferably, the maximum length of the opening 5B is more than 120 mm and in the range of less than 40% of the maximum length of the disposable-diaper main body 10 in the width direction WD. This is because the opening 5B requires a length of more than 100 mm to allow the wearer to draw out his male sex organ. When the length of the opening 5B exceeds the range of 60% of the length of the disposable-diaper main body 10 in the width direction WD, the disposable diaper 1B becomes difficult to pull up during wearing, leading to poor ease of use.

The length of the superimposed portion in the front body 2 is more than 15 mm, for example. Preferably, the length of the superimposed portion is more than 30 mm. The non-joined part 22 includes filiform elastic members 63 in its entirety or in part. The elastic members 63 may be band-like elastic members such as a stretchable nonwoven fabric or a stretchable film.

The openable non-joined part 22 may have a predetermined shape such as a dot shape, a portion of which can be joined in the easily removable state. Specifically, the non-joined part 22 may not be joined in its entirety or may be joined in part by a joining means or device that can be removed easily. The joining method of the junctions 21 includes hotmelt adhesion, heat-sealing, ultrasonic sealing, etc.

For the purpose of easy recognition of the location of the opening 5B, the opening 5B may be colored on the rise direction RD side or leg-direction side of the opening 5B or provide the irregularities by embossing, etc., for example. Thus, the opening 5B may be configured to provide a sign for facilitating recognition of the location of the opening 5B visually or tactually. It is desirable that providing such visual or tactual sign allows easy recognition of the location of the opening 5B when the wearer urinates by himself, for example, leading to easier urination. Moreover, when assisting urination of a bedridden patient, etc., for example, a caregiver can easily recognize the location of the opening 5B of the disposable diaper 1B worn by the patient, etc., leading to facilitation of urination assistance in care.

An opening auxiliary such as a handle may be provided to the opening 5B. It is desirable that providing the opening auxiliary allows the wearer to easily separate the opening 5B using the opening auxiliary, for example. Moreover, this allows easy enlargement of the opening 5 when assisting urination of a bedridden patient, etc. in care, for example.

Preferably, the opening 5B is located in the range of the trunk periphery 23 of the front body 2. Specifically, the opening 5B is arranged at a location of the front body 2 a predetermined distance away from a given outer edge of the absorbent core 12, i.e., one side of the absorbent core 12 extending in the width direction WD. With the location of the opening 5B, assuming that the distance from the end of the trunk opening 9 to the upper end of the chassis 13A is h, the distance from the upper end of the chassis 13A to the longitudinal direction center L in the developed state is i, and the distance from the end of the trunk opening 9 to the longitudinal direction center L in the developed state is j, a value of i/h is, preferably, in the range from 0.65 to 8.0.

Specifically, the distance h from the end of the trunk opening 9 to the upper end of the chassis 13A is, preferably, in the range from 50 mm to 270 mm. The distance i from the upper end of the chassis 13A to the longitudinal direction center L in the developed state is, preferably, in the range from 180 mm to 400 mm. The distance j from the end of the trunk opening 9 to the longitudinal direction center L in the developed state is, preferably in the range from 350 mm to 450 mm.

Preferably, the opening 5B is arranged on the rise direction RD side of the absorbent core 12. Specifically, assuming that the distance from the longitudinal direction end of the absorbent core 12 in the front body 2 to the longitudinal direction center L in the developed state is k, and the distance from the upper end of the chassis 13A to the longitudinal direction end of the absorbent core 12 in the front body 2 is 1, a value l/k is, preferably, in the range from greater than 1.0 to less than 2.3. Specifically, the distance k from the longitudinal direction end of the absorbent core 12 in the front body 2 to the longitudinal direction center L in the developed state is, preferably, in the range from 175 mm to 395 mm. The distance l from the upper end of the chassis 13A to the longitudinal direction end of the absorbent core 12 in the front body 2 is, preferably, in the range from 5 mm to 225 mm.

With the disposable diaper that is smaller than the disposable diaper 1B in this embodiment, i.e., so called disposable diapers for children, the distance h from the end of the trunk opening 9 to the upper end of the chassis 13A is, preferably, in the range from 35 mm to 180 mm, for example. The distance i from the upper end of the chassis 13A to the longitudinal direction center L in the developed state is, preferably, in the range from 120 mm to 265 mm. The distance j from the end of the trunk opening 9 to the longitudinal direction center L in the developed state is, preferably, in the range from 200 mm to 300 mm.

With the absorbent core 12 for children, the distance k from the longitudinal direction end of the absorbent core 12 in the front body 2 to the longitudinal direction center L in the developed state is, preferably, in the range from 115 mm to 260 mm. The distance 1 from the upper end of the chassis 13A to the longitudinal direction end of the absorbent core 12 in the front body 2 is, preferably, in the range from 5 mm to 150 mm.

Referring to FIGS. 18A and 18B, an elastic member is provided, preferably, to at least one of the chassis 13A and 13B adjacent to the opening 5B. In this case, the elastic member may be either filiform or like a sheet. The elastic member may be provided to both of the chassis 13A and 13B adjacent to the opening 5B.

Referring to FIGS. 19 and 20, with the usage mode of the disposable diaper 1B, the wearer catches a substantial center of the chassis 13A of the opening 5B formed in the non-joined part 22 with his fingers, and pulls it in the leg direction LGD. With this, the elastic back body area 55 or part of the opening stretchable area 53 is extended by an elastic force, so that the opening 5B is extended in the leg direction LGD, facilitating securing of the opening of a predetermined size.

Thus, in the front body 2, by superimposing the chassis 13A and 13B one upon another to provide the opening 5B defined by the junctions 21 and the non-joined part 22 and arranging the elastic members 63 at the opening 5B, the opening 5 can be formed, which can be opened as required. When the non-joined part 22 is joined in the dot shape as well, joining can easily be removed, allowing forming of the opening 5B that can be opened as required. No masking sheet 6 is arranged in the second embodiment. Without being limited thereto, the masking sheet 6 may be arranged in the second embodiment as in the first embodiment.

### 2.2. Third embodiment

Referring to FIGS. 21 to 23, a disposable diaper 1C in the third embodiment of the present invention will be described. Referring to FIG. 22, the disposable diaper 1C includes a filiform elastic member or a band-like elastic member such as a stretchable nonwoven fabric or a stretchable film arranged adjacent to the boundary including a joined or engaged position between the front body 2 and the back body 3 to serve as the elastic boundary area 56. With this, on the trunk periphery 23, stress acting on parts of the openable part 5C in the rise direction RD and in the leg direction LGD is reduced as compared with stress acting on other parts. Adjacent to the leg openings 7, the --------------------→ filiform elastic member or the band-like elastic member such as a stretchable nonwoven fabric or a stretchable film may be arranged around the circumference in its entirety or in part, or may not arranged therearound.

Referring to FIG. 21, in the third embodiment, the disposable diaper 1C can use a masking sheet 6C having a single nonwoven fabric 67 of 10 g/m² to 30 g/m² or a lamination of nonwoven fabrics 67. In this case, the masking sheet 6C does not necessarily require an elastic member. When the masking sheet 6C is composed of a plurality of nonwoven fabrics 67, a liquid-impermeable sheet 66 such as a polyethylene (PE) or polypropylene (PP) film may be placed on any one of the nonwoven fabrics 67.

Thus, in the third embodiment, the disposable diaper 1C can maintain the length of the openable part 5C in the worn state close to the maximum length by reducing stress acting on the parts of the openable part 5C in the rise direction RD and in the leg direction LGD as compared with stress acting on other parts. The elastic boundary area 56 or part of the opening stretchable area 53 is extended by an elastic force, so that the openable part 5C is extended in the leg direction LGD, facilitating securing of the opening of a predetermined size.

### 2.3. Fourth embodiment

Referring to FIGS. 24 to 26, a disposable diaper 1D in the fourth embodiment of the present invention will be described. Referring to FIG. 24, the disposable diaper 1D in the fourth embodiment differs from the disposable diaper 1 in the first embodiment in the configuration of forming the opening 5D. Specifically, a rectangular chassis 13D is superimposed upon a chassis 13C by a predetermined amount at a predetermined location of the front body 2 to provide a superimposed portion, and the chassis 13D and 13C are joined together at a pair of junctions 21 or both sides of the superimposed portion, forming the front body 2. The superimposed portion becomes openable by arranging a non-joined part 22 substantially in the center of the front body 2.

The non-joined part 22 may have a predetermined shape such as a dot shape, a portion of which can be joined in the easily removable state. Specifically, the non-joined part 22 may not be joined in its entirety or may include a weakly joined portion that can be removed easily. The joining method of the junctions 21 includes hotmelt adhesion, heat-sealing, ultrasonic sealing, etc.

The length of the superimposed portion in the front body 2 is greater than 15 mm, for example. Preferably, the length of the superimposed portion is greater than 30 mm.

Referring to FIGS. 25 and 26, in the fourth embodiment, the disposable diaper 1D includes a filiform elastic member or a band-like elastic member such as a stretchable nonwoven fabric or a stretchable film arranged adjacent to the boundary including a joined or engaged position between the front body 2 and the back body 3 to serve as the elastic boundary area 56. With this, on the trunk periphery 23, stress acting on parts of the openable part 5C in the rise direction RD and in the leg direction LGD is reduced as compared with stress acting on other parts. Adjacent to the leg openings 7, the filiform elastic member or the band-like elastic member such as a stretchable nonwoven fabric or a stretchable film may be arranged around the circumference in its entirety or in part, or it may not be arranged therearound.

Thus, with the disposable diaper 1D in the fourth embodiment, the chassis 13C and 13D are superimposed one upon another in the front body 2 to arrange the opening 5D defined by the junctions 21 and the non joint 22. The disposable diaper 1D can maintain the length of the opening 5D in the worn state close to the maximum length by reducing stress acting on the parts of the opening 5D in the rise direction RD and in the leg direction LGD as compared with stress acting on other parts. Moreover, the elastic boundary area 56 or part of the opening stretchable area 53 is extended by an elastic force, so that the opening 5D is extended in the leg direction LGD, facilitating securing of the opening of a predetermined size. Additionally, when the non-joined part 22 is weakly joined to the dot shape area, joining can easily be removed allowing the forming of the opening 5D that can be opened as required.

The trunk periphery 23 may be formed with a substrate sheet, which can be stretched, in place of an elastic member.

### 3. Components

The components of the disposable diaper 1 will be described hereafter.

### 3.1. Main body of the disposable diaper

### 3.1.1. Top sheet

The top sheet 11 is arranged on the wearer's body side during use to make contact with excrement. The top sheet 11 may be liquid permeable over the entire surface or part thereof. The top sheet 11 may be composed of either a single sheet-like member or a plurality of sheet-like members bonded together.

Preferably, the top sheet 11 is made of a material having a strength that can resist damage even when undergoing a load due to compression, twist, friction, etc. during wearing and providing no stimulus to the skin, for example. A sheet-like material having liquid permeability can be adopted such as a fabric, a nonwoven fabric or a perforated plastic sheet, for example. An example of the nonwoven fabric is a fabric made of hydrophobic fibers of polyolefin or polyester subjected to hydrophilic treatment and manufactured by pointbond, airthrough, spunbond or the like. Examples of materials of the nonwoven fabric are natural and chemical fibers having a basis weight of 15 g/m² to 35 g/m². An example of natural fibers is cellulose such as cotton. Examples of chemical fibers are semi-synthetic cellulose such as rayon, regenerated cellulose, acetate or triacetate, thermoplastic hydrophobic chemical fiber, and thermoplastic hydrophobic chemical fiber subjected to hydrophilic treatment. Examples of thermoplastic hydrophobic chemical fibers are single fiber made of polyethylene (PE), polypropylene (PP) or polyethylene terephthalate (PET), fiber obtained by graft polymerization of polyethylene (PE) and polypropylene (PP), and composite fiber having core/sheath structure or the like.

Examples of the perforated plastic sheet are perforated sheets of thermoplastic resin such as polyethylene (PE), polypropylene (PP) or polyethylene terephthalate (PET) and a perforated sheet of a porous foaming material.

### 3.1.2. Absorbent core

The absorbent core 12 serves to absorb and hold discharged bodily fluids such as urine. Examples of the absorbent core are a mixed laminate of high absorbent polymer and hydrophilic fibers and a structure having high absorbent polymer fixed to hydrophilic sheets with a hotmelt adhesive, etc. An example of the high absorbent polymer is an absorbent polymer such as polyacrylate or starch acrylate, wherein the components having a water absorption capacity of 20 g/g or more and a particle size of 100 µm to 800 µm constitute 80% or more. Examples of the hydrophilic fibers are crushed pulp fibers and rayon fibers. Examples of the hydrophilic sheets are sheets made of nonwoven fabrics having a basis weight of 15 g/m² to 35 g/m² and obtained from hydrophobic fibers of polyolefin, polyester, etc. subjected to hydrophilic treatment, the sheets being manufactured by pointbond, airthrough, spunbond or the like. An example of the hotmelt adhesive is an adhesive containing as a base polymer a styrene-butadiene-styrene copolymer (SBS), a styrene-isobutylene-styrene copolymer (SIS), a styrene- ethylene-butylene-styrene copolymer (SEBS), a styrene-ethylene-propylene-styrene copolymer (SEPS), an amorphous poly-alpha-olefin (APAO) or the like.

### 3.1.3. Chassis

The chassis 13 provides an outer shape of the main body 10 of the disposable diaper 1. The chassis 13 may be composed of either a single sheet-like member or a plurality of sheet-like members bonded together. In the first embodiment, the disposable diaper 1 is configured so that the chassis 13 includes a front body 2, a back body 3, and a crotch 4, wherein the top sheet 11, absorbent core 12, and back sheet 14 are arranged at predetermined locations of the chassis 13. Without being limited thereto, the chassis 13 may include the front body 2 and the back body 3, wherein the crotch 4 having the top sheet 11, absorbent core 12, and back sheet 14 may independently be joined to the chassis 13. In this alternative, the back sheet 14 and the chassis 13 may be joined at the crotch 4, and the back sheet 14 may be configured to play a role of the chassis 13.

Preferably, the chassis 13 is made of a material having a strength that can resist damage even when undergoing a load due to compression, twist, friction, etc. during wearing and providing no stimulus to the skin, for example. A sheet-like material having gas permeability can be adopted such as a nonwoven fabric or a perforated plastic sheet, for example. An example of the nonwoven fabric is a fabric made of synthetic fibers of polyolefin or polyester and manufactured by pointbond, airthrough, spunbond or the like. Examples of materials of the nonwoven fabric are natural and chemical fibers having basis weight of 15 g/m² to 35 g/m². An example of natural fibers is cellulose such as cotton. Examples of chemical fibers are semi-synthetic cellulose such as rayon, regenerated cellulose, acetate or triacetate, thermoplastic hydrophobic chemical fiber, and thermoplastic hydrophobic chemical fiber subjected to hydrophilic treatment. Examples of thermoplastic hydrophobic chemical fibers are single fiber made of polyethylene (PE), polypropylene (PP) or polyethylene terephthalate (PET), fiber obtained by graft polymerization of polyethylene (PE) and polypropylene (PP), and composite fiber having core/sheath structure or the like.

Examples of the perforated plastic sheet are a perforated sheet of thermoplastic resin such as polyethylene (PE), polypropylene (PP) or polyethylene terephthalate (PET) and a perforated sheet of a porous foaming material.

### 3.1.4. Back sheet

Examples of the back sheet 14 are a thermoplastic film made principally of polyethylene (PE), polypropylene (PP) or the like, a resin film having gas permeability, and a composite obtained by joining a resin film having gas permeability to a nonwoven fabric manufactured by spunbond or spunlace. A resin film is desirable, which is made principally of a resin of polyolefin and has basis weight of 10 g/m² to 30 g/m², for example.

### 3.1.5. Elastic member

Examples of materials of the elastic member 91 are natural rubber and synthetic rubber made of styrene-butadiene, butadiene, isoprene or the like. An example of the sheet-like elastic member is a nonwoven fabric obtained from mixed fibers of a polyurethane foam having a weight of 30 g/m² to 80 g/m², polyurethane having a weight of 20 g/m² to 70 g/m², and polypropylene.

### 3.1.6. Adhesive

The top sheet 11 and absorbent core 12 are adhesively joined to the chassis 13 with the hotmelt adhesive. The top sheet 11 and the absorbent core 12 are adhesively joined with the hotmelt adhesive. Without being limited to hotmelt adhesion, joining may be made by heat-sealing, ultrasonic sealing, etc. alone or in combination.

An example of the hotmelt adhesive is an adhesive containing as a base polymer a styrene-butadiene-styrene copolymer (SBS), a styrene-isobutylene-styrene copolymer (SIS), a styrene- ethylene-butylene-styrene copolymer (SEBS), a styrene-ethylene-propylene-styrene copolymer (SEPS), an amorphous poly-alpha-olefin (APAO) or the like. The coating patterns when joining is made by hotmelt adhesion include spiral coating, controlled seam coating, coater coating, curtain coater coating, summit-gun coating, etc., for example. The weight of an adhesive for hotmelt adhesion ranges, preferably, from 1 g/m² to 30 g/m², and more preferably, from 3 g/m² to 10 g/m². With the pattern having an adhesive coated linearly, the line diameter ranges, preferably, from 30 µm to 300 µm.

### 3.2. Masking sheet

### 3.2.1. Substrate sheet

The substrate sheets 61 and 62 can be either a liquid-permeable sheet or a liquid-impermeable sheet. When adopting the liquid-permeable sheet, the liquid-permeable sheet illustrated as the top sheet 11 can be used, for example. The substrate sheets 61 and 62 may be a thin nonwoven fabric simply, such as a spunbond nonwoven fabric, pointbond nonwoven fabric or spunlace nonwoven fabric, the basis weight of which ranges from 10 g/m² to 30 g/m². This is because a thinner nonwoven fabric provides enhanced smoothness of the band-like portion in the stretched state.

When adopting the liquid-impermeable sheet, the liquid-impermeable sheet illustrated as the back sheet 14 can be used, for example. The liquid-impermeable sheet may be provided to the elastic member either on the skin contact side or on the skin non-contact side.

### 3.2.2. Elastic member

Examples of the elastic members 63 are members made of natural rubber or synthetic rubber such as styrene-butadiene, butadiene, isoprene or the like. An example of the sheet-like elastic member is a nonwoven fabric obtained from mixed fibers of a polyurethane foam having a basis weight of 30 g/m² to 80 g/m², polyurethane having a weight of 20 g/m² to 70 g/m², and polypropylene.

### 3.2.3. Adhesive

With joining of the substrate sheets 61 and 62 and the elastic members 63 or joining of the substrate sheets 61 and 62, when joining the substrate sheets 61 and 62 with a hotmelt adhesive, the hotmelt adhesive is coated thereon by the coating method such as spiral coating, controlled seam coating, coater coating, curtain coater coating, summit-gun coating or the like. The elastic members 63 are superimposed upon the hotmelt adhesive, upon which the substrate sheets 61 and 62 are superimposed for joining. In order to obtain hard disengagement of the elastic members 63 from the substrate sheets 61 and 62, the hotmelt adhesive may be coated in advance by the coating method such as slit coating, controlled seam coating or the like. Without being limited to hotmelt adhesion, joining may be made by heat-sealing, ultrasonic sealing, etc. alone or in combination.

An example of the hotmelt adhesive is an adhesive containing as a base polymer a styrene-butadiene-styrene copolymer (SBS), a styrene-isobutylene-styrene copolymer (SIS), a styrene- ethylene-butylene-styrene copolymer (SEBS), a styrene-ethylene-propylene-styrene copolymer (SEPS), an amorphous poly-alpha-olefin (APAO) or the like. The weight of an adhesive for hotmelt adhesion ranges, preferably, from 1 g/m² to 30 g/m², and more preferably, from 3 g/m² to 10 g/m². With the pattern having an adhesive applied linearly, the line diameter ranges, preferably, from 30 µm to 300 µm.

## Claims

1. A disposable diaper, comprising:
a chassis (13) including at least a front body (2) and a back body (3);
a top sheet (11) arranged on at least part of the chassis, the top sheet being liquid permeable;
a back sheet (14) arranged on the top sheet at one side in a thickness direction of the top sheet, the back sheet being liquid impermeable; and
an absorbent core (12) arranged between the top sheet and the back sheet, the absorbent core being liquid retainable, wherein
the front body (2) and the back body (3) are joined and secured together at a predetermined location in advance or upon wearing the disposable diaper to provide a pants shape with a trunk opening (9) and a pair of leg openings (7),
the front body (2) is formed with one of an openable part (5) or an opening (5), the openable part and the opening each being formed by connecting two points positioned at a distance from each other across a substantial center line extending in a longitudinal direction (LD) of the disposable diaper and bisecting a width direction (WD) thereof, the openable part (5) allowing the front body (2) to be separated at a predetermined site, and the opening (5) allowing the front body (2) to open at a predetermined site,
wherein an opening stretch area (53) is defined to connect a substantially central portion of the back body (3) adjacent to the trunk opening (9) and a predetermined location of the front body (2) adjacent to at least one of the openable part (5) or the opening (5) along the outer perimeter of the pants shape, at least part of the opening stretch area (53) being able to expand and contract, and
wherein the opening stretch area (53) includes an elastic opening area (54) arranged at a predetermined location on the side of the openable part (5) or the opening (5) in a leg direction (LGD) of the pants shape.

2. The disposable diaper according to claim 1, wherein the absorbent core (12) is vertically long and is provided to at least the front body (2), wherein one of the openable part (5) and the opening (5) each are formed in the front body at a location away from a given outer edge of the absorbent core (12).

3. The disposable diaper according to claim 1 or 2, wherein the opening stretch area (53) includes an elastic back body area (55) arranged in at least part of the back body (3) adjacent to the trunk opening (9).

4. The disposable diaper according to any one of claims 1 to 3, wherein the opening stretch area includes an elastic boundary area (56) arranged in at least part of a boundary between the front body (2) and the back body (3), the boundary including at least one of a joined position and an engaged position of the front body (2) and the back body (3).

5. The disposable diaper according to any one of claims 1 to 4, further comprising an auxiliary sheet (6) provided to the front body (2) at a location including at least one of the openable part (5) and the opening (5).

6. The disposable diaper according to claim 5, wherein the auxiliary sheet (6) includes an elastic member at least in part.

7. The disposable diaper according to claim 5 or 6, wherein the auxiliary sheet (6) is arranged with an absorbent core (12) at least in part.

## Patentansprüche

1. Einwegwindel, umfassend:
einen Rahmen (13) mit mindestens einem vorderen Körperteil (2) und einem rückwärtigen Körperteil (3);
eine auf mindestens einem Teil des Rahmens angeordnete Oberlage (11), die flüssigkeitsdurchlässig ist;
eine auf der Oberlage auf einer Seite in Dickenrichtung derselben angeordnete rückwärtige Lage (14), die flüssigkeitsundurchlässig ist; und
einen zwischen der Oberlage und der rückwärtigen Lage angeordneten saugfähigen Kern (12), der Flüssigkeit zurückhalten kann, wobei
das vordere Körperteil (2) und das rückwärtige Körperteil (3) an einer vorgegebenen Stelle im Voraus oder beim Tragen der Einwegwindel zur Herstellung einer Hosenform mit einer Körperöffnung (9) und einem Paar von Beinöffnungen (7) miteinander verbunden und aneinander befestigt werden,
das vordere Körperteil (2) mit einem zu öffnenden Teil (5) oder einer Öffnung (5) versehen ist, die jeweils durch Verbinden von zwei Stellen gebildet sind, die beabstandet von einander quer über eine wesentliche Mittellinie angeordnet sind, die in einer Längsrichtung (LD) der Einwegwindel verläuft und eine Breitenrichtung (WD) derselben halbiert, wobei das zu öffnende Teil (5) das Abtrennen des vorderen Körperteils (2) an einer vorgegebenen Stelle ermöglicht und die Öffnung (5) das Öffnen des vorderen Körperteils (2) an einer vorgegebenen Stelle ermöglicht,
wobei ein Öffnungsdehnungsbereich (53) dazu definiert ist, eine im Wesentlichen mittlere Partie des rückwärtigen Körperteils (3) neben der Körperöffnung (9) mit einer vorgegebenen Stelle des vorderen Körperteils (2) neben dem zu öffnenden Teil (5) und/oder der Öffnung (5) entlang dem Außenumfang der Hosenform zu verbinden, wobei mindestens ein Teil des Öffnungsdehnungsbereichs (53) ausdehnbar und zusammenziehbar ist, und
wobei der Öffnungsdehnungsbereich (53) einen elastischen Öffnungsbereich (54) umfasst, der an einer vorgegebenen Stelle auf der Seite des zu öffnenden Teils (5) oder der Öffnung (5) in einer Beinrichtung (LGD) der Hosenform angeordnet ist.

2. Einwegwindel nach Anspruch 1, wobei der saugfähige Kern (12) senkrecht lang ist und für mindestens das vordere Körperteil (2) vorgesehen ist, wobei das öffnende Teil (5) oder die Öffnung (5) im vorderen Körperteil an einer Stelle ausgebildet ist, die von einem gegebenen Außenrand des saugfähigen Kerns (12) entfernt ist.

3. Einwegwindel nach Anspruch 1 oder 2, wobei der Öffnungsdehnungsbereich (53) einen elastischen rückwärtigen Körperteilbereich (55) umfasst, der in mindestens einem Teil des rückwärtigen Körperteils (3) neben der Körperöffnung (9) angeordnet ist.

4. Einwegwindel nach einem der Ansprüche 1 bis 3, wobei der Öffnungsdehnungsbereich einen elastischen Grenzbereich (56) umfasst, der in mindestens einem Teil einer Grenze zwischen dem vorderen Körperteil (2) und dem rückwärtigen Körperteil (3) angeordnet ist, wobei die Grenze eine vorbundene Stelle und/oder eine Eingriffstelle des vorderen Körperteils (2) und des rückwärtigen Körperteils (3) umfasst.

5. Einwegwindel nach einem der Ansprüche 1 bis 4, weiter umfassend eine Zusatzlage (6), die für das vordere Körperteil (2) an einer Stelle vorgesehen ist, die das zu öffnende Teil (5) und/oder die Öffnung (5) umfasst.

6. Einwegwindel nach Anspruch 5, wobei die Zusatzlage (6) mindestens teilweise ein elastisches Element umfasst.

7. Einwegwindel nach Anspruch 5 oder 6, wobei die Zusatzlage (6) mindestens teilweise mit einem saugfähigen Kern (12) ausgestattet ist.

## Revendications

1. Couche jetable, comportant :
une armature (13) comprenant un corps avant (2) et un corps arrière (3) ;
une feuille de dessus (11) agencée sur au moins une partie de l'armature, la feuille de dessus étant perméable aux liquides ;
une feuille de support (14) agencée sur la feuille de dessus au niveau d'un côté dans une direction allant dans le sens de l'épaisseur de la feuille de dessus, la feuille de support étant imperméable aux liquides ; et
une partie centrale absorbante (12) agencée entre la feuille de dessus et la feuille de support, la partie centrale absorbante étant en mesure de retenir les liquides, dans laquelle
le corps avant (2) et le corps arrière (3) sont joints et assujettis ensemble au niveau d'un emplacement prédéterminé à l'avance ou lors du port de la couche jetable pour la mise en oeuvre d'une forme de culotte avec une ouverture de slip (9) et une paire d'ouvertures pour les jambes (7),
le corps avant (2) est formé avec l'une parmi une partie ouvrable (5) ou une ouverture (5), la partie ouvrable et l'ouverture étant chacune formées en reliant deux points positionnés à une distance l'un par rapport à l'autre en travers d'une ligne centrale importante s'étendant dans une direction allant dans le sens longitudinal (LD) de la couche jetable et bissectant une direction allant dans le sens de la largeur (WD) de celle-ci, la partie ouvrable (5) permettant au corps avant (2) d'être séparé au niveau d'un site prédéterminé, et l'ouverture (5) permettant au corps avant (2) de s'ouvrir au niveau d'un site prédéterminé,
dans laquelle une zone extensible d'ouverture (53) est définie pour relier une partie sensiblement centrale du corps arrière (3) se trouvant de manière adjacente par rapport à l'ouverture de slip (9) et un emplacement prédéterminé du corps avant (2) se trouvant de manière adjacente par rapport à au moins l'une parmi la partie ouvrable (5) ou l'ouverture (5) le long du périmètre extérieur de la forme de culotte, au moins une partie de la zone extensible d'ouverture (53) étant en mesure de s'étendre et de se contracter, et
dans laquelle la zone extensible d'ouverture (53) comprend une zone d'ouverture élastique (54) agencée au niveau d'un emplacement prédéterminé sur le côté de la partie ouvrable (5) ou l'ouverture (5) dans une direction allant dans le sens des jambes (LGD) de la forme de culotte.

2. Couche jetable selon la revendication 1, dans laquelle la partie centrale absorbante (12) est longue à la verticale et est mise en oeuvre sur au moins le corps avant (2), dans laquelle l'une parmi la partie ouvrable (5) et l'ouverture (5) est formée dans le corps avant au niveau d'un emplacement à distance d'un bord extérieur donné de la partie centrale absorbante (12).

3. Couche jetable selon la revendication 1 ou la revendication 2, dans laquelle la zone extensible d'ouverture (53) comprend une zone de corps arrière élastique (55) agencée dans au moins une partie du corps arrière (3) se trouvant de manière adjacente par rapport à l'ouverture de slip (9).

4. Couche jetable selon l'une quelconque des revendications 1 à 3, dans laquelle la zone extensible d'ouverture comprend une zone limite élastique (56) agencée dans au moins une partie d'une limite entre le corps avant (2) et le corps arrière (3), la limite comprenant au moins l'une parmi une position jointe et une position mise en prise du corps avant (2) et du corps arrière (3).

5. Couche jetable selon l'une quelconque des revendications 1 à 4, comportant par ailleurs une feuille auxiliaire (6) mise en oeuvre sur le corps avant (2) au niveau d'un emplacement comprenant au moins l'une parmi la partie ouvrable (5) et l'ouverture (5).

6. Couche jetable selon la revendication 5, dans laquelle la feuille auxiliaire (6) comprend un élément élastique au moins en partie.

7. Couche jetable selon la revendication 5 ou la revendication 6, dans laquelle la feuille auxiliaire (6) est agencée avec une partie centrale absorbante (12) au moins en partie.
